(19) Europäisches Patentamt
European Patent Office
Office européen des brevets

(11) **EP 4 338 682 A2**

(12) **EUROPEAN PATENT APPLICATION**

(43) Date of publication:
**20.03.2024 Bulletin 2024/12**

(21) Application number: **23193778.0**

(22) Date of filing: **28.08.2023**

(51) International Patent Classification (IPC):
**A61B 8/08** (2006.01)    **A61B 5/02** (2006.01)
**A61B 5/00** (2006.01)    **A61B 8/00** (2006.01)
**G16H 50/30** (2018.01)

(52) Cooperative Patent Classification (CPC):
**A61B 8/0891; A61B 5/02007; A61B 5/7267;
A61B 8/0858; A61B 8/4427; A61B 8/5223;
G16H 50/30**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **26.08.2022  US 202263401387 P**

(71) Applicant: **SightIn Health SA**
**3963 Crans-Montana (CH)**

(72) Inventors:
• **JATAUTAS, Marius**
  **1201 Geneva (CH)**
• **ARDERIU, Andreu Romero**
  **1007 Lausanne (CH)**
• **CASTELLANOS, Pablo Cañas**
  **1004 Lausanne (CH)**
• **NAMDAR, Mehdi**
  **1299 Crans (CH)**
• **KING IV, William Frederick**
  **1223 Cologny (CH)**

(74) Representative: **Lecomte & Partners**
**76-78, rue de Merl**
**2146 Luxembourg (LU)**

(54) **METHODS, SYSTEMS, DEVICES AND COMPONENTS FOR RAPID ON-SITE MEASUREMENT, CHARACTERIZATION AND CLASSIFICATION OF A PATIENT'S CAROTID ARTERY USING A PORTABLE ULTRASOUND PROBE OR DEVICE**

(57) Described and disclosed herein are various embodiments of methods and systems configured to rapidly measure, analyze and provide, in for example an on-site and out-patient setting within a short period of time, one or more physical parameters associated with a one or more of a patient's carotid arteries. Some embodiments comprise at least one computing device, at least one portable handheld ultrasound device or probe operably connected to the at least one computing device, the portable handheld ultrasound device being configured to provide to the computing device as outputs therefrom a series of ultrasound image frames acquired from the one or more of the patient's carotid arteries, and a display or monitor operably connected to the at least one computing device and configured to visually display to a user results generated by the at least one computing device. Among other things, the ultrasound image frames are processed to guide a clinician in accurately, quickly and efficiently placing the ultrasound probe on the patient's neck and body so that the physical characteristics of a patient's carotid arteries can be detected and measured.

EP 4 338 682 A2

Fig. 1

## Description

## Field of the invention

The present invention relates to systems, devices, components and methods for rapid on-site characterization and classification of a patient's carotid artery, which in some embodiments comprises processing brightness mode ultrasound images in medical applications for the measurement of cardiovascular parameters.

## Background

Carotid intima-media thickness (cIMT) testing via brightness mode (B-mode) ultrasound is a known method for evaluating cardiovascular risk by measuring the thickness of the intimal and medial layers of the artery wall. It advantageously allows non-invasive ultrasonic detection of carotid plaque and cIMT measurements that may be used to assess cardiovascular risks in a patient and detect possible vascular disease. The medical practitioner typically uses a portable ultrasound device to scan a patient's carotid artery, and based on the captured images, may perform a diagnosis including a measurement of the cIMT, plaque localization, plaque volume, maximum plaque height, plaque echogenicity, intraplaque neovascularization, stenosis progression and arterial stiffness in combination with a blood measurement device. These various assessments may be used to evaluate an individual's cardiovascular risk.

Accurate and reliable assessment of pictorial information by a skilled sonographer may vary depending on multiple factors including the level of skill of the sonographer, the morphology of the patient, and the precision of the instruments used. There may be quite a large variability in the diagnosis based on the pictorial assessments made by the practitioner. There would therefore be need to provide a more reliable diagnosis and a system to assist the practitioner in performing the assessment and reading the results.

## Summary

Various embodiments provide a system for evaluating cardiovascular health parameters using portable brightness mode ultrasound devices that generates reliable and reproduceable results and that assists the healthcare practitioner in performing an accurate diagnosis.

According to one embodiment, there is provided a system configured to rapidly measure, analyze and provide, in an on-site or out-patient setting and within a predetermined period of time, one or more physical parameters or characteristics associated with a one or more of a patient's carotid arteries, where the system comprises at least one computing device; at least one portable ultrasound device or probe operably connected to the at least one computing device, the portable ultrasound device being configured to provide to the computing device as outputs therefrom a series of ultrasound image frames acquired from the one or more of the patient's carotid arteries, and a display or monitor operably connected to the at least one computing device and configured to visually display to a user results generated by the at least one computing device; wherein the computing device comprises at least one non-transitory computer readable medium configured to store instructions executable by at least one processor to process the ultrasound image frames, the at least one processor and the at least one non-transitory computer readable medium further being configured to process the ultrasound image frames using a trained discriminative or convolutional machine learning model, the computing device and ultrasound device or probe being configured to: (i) identify at least one of a bifurcation, a transverse view, and a cross-sectional view of at least one of the patient's carotid arteries from among the ultrasound image frames generated by the ultrasound device or probe; (ii) generate directional and locational instructions to the user on the display or monitor or via sound regarding the location, placement, orientation and movement of the ultrasound device or probe; (iii) measure and compute at least one of lumen distention, carotid intima-media thickness (cIMT), cardiovascular age, plaque characterization, and local arterial stiffness of the at least one carotid artery; and (iv) provide as outputs from the computing device at least one of the measured and computed lumen distention, the measured and computed carotid intima-media thickness (cIMT), the measured and computed cardiovascular age, the measured and computed plaque characterization, and the measured and computed local arterial stiffness of the at least one carotid artery to one or more of the monitor or display, a printer, a speaker or headphones, or data formatted for digital or memory storage or transmission.

Such an embodiment may further comprise one or more of: (a) the ultrasound device or probe being configured to switchably and controllably operate in B-mode or M-mode; (b) the ultrasound device or probe being configured to switchably and controllably operate in the M-mode when measuring lumen distension; (c) the trained discriminative or convolutional machine learning model being trained and configured to generate directional and locational instructions to the user on the display or monitor or via sound regarding at least one of the location, placement, orientation and movement of the ultrasound device or probe to identify one or more of carotid artery bifurcation locations, carotid artery cross-sectional characteristics, carotid artery plaque characteristics, carotid artery lumen characteristics, carotid artery regions of interest, and carotid artery far wall variability; (d) the trained discriminative or convolutional machine learning model is trained and configured to provide directional and locational instructions to the user regarding whether the ultrasound probe or device is centered over the patient's carotid artery; (e) lumen distension being measured and computed using the patient's blood pressure as an input; (f) the trained discriminative or convolutional machine learning model being trained and configured to crop the ultrasound image frames so as to enhance reliability of the detection and identification one or more of carotid artery bifurcation locations, carotid artery cross-sectional characteristics, carotid artery plaque characteristics, carotid artery lumen characteristics, carotid artery regions of interest, and carotid artery far wall variability; (g) the trained discriminative or convolutional machine learning model being trained and configured to determine the quality of the ultrasound image frames so as to enhance reliability of the detection and identification one or more of carotid artery bifurcation locations, carotid artery cross-sectional characteristics, carotid artery plaque characteristics, carotid artery lumen characteristics, carotid artery regions of interest, and carotid artery far wall variability; (h) the trained discriminative or convolutional machine learning model being trained and configured to measure and compute one or more of the number, location, length, height, area and echogenicity of each plaque in the at least one carotid artery as part of the plaque characterization; and (i) the predetermined period of time being about ten minutes or less.

In another embodiment, there is provided a method of rapidly measuring, analyzing and providing, in an on-site and out-patient setting within a predetermined period of time, one or more physical parameters associated with a one or more of a patient's carotid arteries, the system comprising at least one computing device, at least one portable handheld ultrasound device or probe operably connected to the at least one computing device, the portable handheld ultrasound device being configured to provide to the computing device as outputs therefrom a series of ultrasound image frames acquired from one or more of the patient's carotid arteries, and a display or monitor operably connected to the at least one computing device and configured to visually display to a user results generated by the at least one computing device, the computing device comprising at least one non-transitory computer readable medium configured to store instructions executable by at least one processor to process the ultrasound image frames, the at least one processor and the at least one non-transitory computer readable medium further being configured to process the ultrasound image frames using a trained discriminative or convolutional machine learning model, using the computing device, ultrasound device and the display or monitor, the method comprising: (i) identifying at least one of a bifurcation, a transverse view, and a cross-sectional view of at least one of the patient's carotid arteries from among the ultrasound image frames generated by the ultrasound device or probe; (ii) generating directional and locational instructions to the user on the display or monitor or via sound regarding the location, placement, orientation and movement of the ultrasound device or probe; (iii) measuring and computing at least one of lumen distension, carotid intima-media thickness (cIMT), cardiovascular age, plaque characterization, and local arterial stiffness of the at least one carotid artery; and (iv) providing as outputs from the computing device at least one of the measured and computed lumen distension, the measured and computed carotid intima-media thickness (cIMT), the measured and computed cardiovascular age, the measured and computed plaque characterization, and the measured and computed local arterial stiffness of the at least one carotid artery to one or more of the monitor or display, a printer, a speaker or headphones, or data formatted for digital or memory storage or transmission.

Such an embodiment may further comprise one or more of: (a) the ultrasound device or probe being configured to switchably and controllably operate in B-mode or M-mode; (b) the ultrasound device or probe being configured to switchably and controllably operate in the M-mode when measuring lumen distension; (c) the trained discriminative or convolutional machine learning model being trained and configured to generate directional and locational instructions to the user on the display or monitor or via sound regarding at least one of the location, placement, orientation and movement of the ultrasound device or probe to identify one or more of carotid artery bifurcation locations, carotid artery cross-sectional characteristics, carotid artery plaque characteristics, carotid artery lumen characteristics, carotid artery regions of interest, and carotid artery far wall variability; (d) the trained discriminative or convolutional machine learning model is trained and configured to provide directional and locational instructions to the user regarding whether the ultrasound probe or device is centered over the patient's carotid artery; (e) lumen distension being measured and computed using the patient's blood pressure as an input; (f) the trained discriminative or convolutional machine learning model being trained and configured to crop the ultrasound image frames so as to enhance reliability of the detection and identification one or more of carotid artery bifurcation locations, carotid artery cross-sectional characteristics, carotid artery plaque characteristics, carotid artery lumen characteristics, carotid artery regions of interest, and carotid artery far wall variability; (g) the trained discriminative or convolutional machine learning model being trained and configured to determine the quality of the ultrasound image frames so as to enhance reliability of the detection and identification one or more of carotid artery bifurcation locations, carotid artery cross-sectional characteristics, carotid artery plaque characteristics, carotid artery lumen characteristics, carotid artery regions of interest, and carotid artery far wall variability; (h) the trained discriminative or convolutional machine learning model being trained and configured to measure and compute one or more of the number, location, length, height, area and echogenicity of each plaque in the at least one carotid artery as part of the plaque characterization; and (i) the predetermined period of time being about ten minutes or less.

In still another embodiment, there is provided a system for processing brightness mode ultrasound images in medical applications, in particular for measurement of cardiovascular parameters, comprising a computing system and program modules executable in the computing system configured to process data generated by a B-mode ultrasound probe device of a patient's carotid artery, the program modules include modules for processing an image generated by the B-mode ultrasound probe device, including automated frame cropping of said image, lumen delineation, far wall ROI segmentation and cIMT delineation, wherein the lumen delineation module is configured to: divide said image in a plurality of columns extending from a first side of the image captured closest to the ultrasound probe device to a second opposite side of the image captured furthest from the ultrasound probe device; determine in each column at least a first and a last significant local intensity maxima 11, I2, I3 of image pixels above a chosen fixed intensity threshold; determine in each column at least one significant local intensity minima i1, i2 of image pixels between the first significant local intensity maxima 11 and the last significant local intensity maxima I3; compute paths of connected pixels from said local significant local intensity minima i1, i2 within a predefined distance from the significant local intensity maxima; calculate the lengths of the paths; select paths having a length greater than a fixed proportion of a path with the greatest length, said fixed proportion in a range of 0,7 to 0,95, and define a lumen axis as the selected path furthest from the first side of the image.

Such an embodiment may further comprise one or more of: (a) the intensity value of image pixels being normalized in a fixed scale, for example from 0 to 1; (b) fixed intensity threshold being in a range of 0.1 to 0.3 of the fixed scale, preferably in a range of 0.15 to 0.25; (c) three said significant local intensity maxima 11, I2, I3 of image pixels above a chosen fixed intensity threshold being determined, and two said significant local intensity minima i1, i2 being determined; (d) said predefined distance from the significant local intensity maxima falls within a predetermined range; (e) the frame cropping module being configured to generate a binary mask in which pixels whose intensity values lie between a first intensity threshold T1 and a second intensity threshold T2 are set to bright, and pixels having an intensity below the first threshold and above the second threshold are set to dark, and wherein the frame cropping or lumen delineation module is configured to apply a soothing filter, for example a gaussian smoothing filter, to the binary mask for further processing by the lumen delineation module; (f) the far wall ROI segmentation and cIMT delineation modules being configured to generate a binary mask of the image after the lumen delineation processing in which a threshold T is computed to separate pixels into two classes according to their brightness intensity: - pixels having intensities below the threshold set to dark pixels and corresponding to the lumen; - pixels having intensities above the threshold set to bright and corresponding to the wall interfaces below the lumen and other hyperechoic tissues; and identify a far wall line of the artery in columns extending from the first side of the image to the second opposite side of the image, as the first line of bright pixels extending laterally across the columns; (g) the far wall ROI segmentation and cIMT delineation modules being configured to - smooth the far wall line with a smoothing filter, for example a Savitzky-Golay filter, and - define upper and lower bounds of a far wall ROI with a predefined height parameter to generate a far wall ROI image; (h) the cIMT delineation module is configured to - apply a filter, for example a gaussian filter, on the far wall ROI image to reduce speckle noise and generate a smoothed intensity map, - apply a Sobel filter on the smoothed intensity map in the column direction $y$ to retrieve a $y$-gradient map of a region of interest, - retrieve continuous paths passing through maximum intensity values defined as a center of adventitia layer, - retrieve all gradient local maxima located above the adventitia center, - select a pre-defined number of the longest paths and retaining overlapping paths, which are considered as lumen-intima or media-adventitia candidates, the paths located closest to the first side being identified as lumen-intima, and the paths closest to the second side being identified as the media-adventitia, - define as ROI a section having a pair of paths with the largest overlap; and (i) the program modules comprise a transverse carotid and bifurcation identification module to provide visual feedback to assist a medical practitioner in identifying a carotid artery and a bifurcation of the carotid artery, the transverse carotid and bifurcation identification module configured to identify and mark with a bounding box the carotid in a transverse B-mode ultrasound image, and identify the bifurcation if a ratio of a length to height of the bounding box is above a certain fixed threshold.

In yet another embodiment, there is provided a method of processing brightness mode ultrasound images in medical applications, in particular for measurement of cardiovascular parameters, comprising executing program modules in a computing system configured to process data generated by a B-mode ultrasound probe device of a patient's carotid artery, the program modules include modules for processing an image generated by the B-mode ultrasound probe device, including automated frame cropping of said image, lumen delineation, far wall ROI segmentation and cIMT delineation, wherein the lumen delineation module performs the following steps: divide said image in a plurality of columns extending from a first side of the image captured closest to the ultrasound probe device to a second opposite side of the image captured furthest from the ultrasound probe device; - determine in each column at least a first and a last significant local intensity maxima 11, I2, I3 of image pixels above a chosen fixed intensity threshold, - determine in each column at least one significant local intensity minima i1, i2 of image pixels between the first significant local intensity maxima I1 and the last significant local intensity maxima I3, - compute paths of connected pixels from said local significant local intensity minima i1, i2 within a predefined distance from the significant local intensity maxima, - calculate the lengths of the paths, - select paths having a length greater than a fixed proportion of a path with the greatest length, said fixed proportion in a range of 0,7 to 0,95, and - define a lumen axis as the selected path furthest from the first side of the image.

Such an embodiment may further comprise one or more of: (a) the intensity value of image pixels being normalized on a fixed scale, for example from 0 to 1 and the fixed intensity threshold being in a range of 0.1 to 0.3 of the fixed scale, preferably in a range of 0.15 to 0.25; (b) three said significant local intensity maxima 11, I2, I3 of image pixels above a chosen fixed intensity threshold being determined, and two said significant local intensity minima i1, i2 being determined; (c) said predefined distance from the significant local intensity maxima falls within a predetermined range; (d) the frame cropping module - generates a binary mask in which pixels whose intensity values lie between a first intensity threshold T1 and a second intensity threshold T2 are set to bright, and pixels having an intensity below the first threshold and above the second threshold are set to dark, and the frame cropping or lumen delineation module - applies a soothing filter, for example a gaussian smoothing filter, to the binary mask for further processing by the lumen delineation module; (d) the far wall ROI segmentation and cIMT delineation modules - generate a binary mask of the image after the lumen delineation processing in which a threshold T is computed to separate pixels into two classes according to their brightness intensity: - pixels having intensities below the threshold set to dark pixels and corresponding to the lumen, - pixels having intensities above the threshold set to bright and corresponding to the wall interfaces below the lumen and other hyperechoic tissues - identify a far wall line of the artery in columns extending from the first side of the image to the second opposite side of the image, as the first line of bright pixels extending laterally across the columns. (e) the far wall ROI segmentation and cIMT delineation modules - smooth the far wall line with a smoothing filter, for example a Savitzky-Golay filter, and - define upper and lower bounds of a far wall ROI with a predefined height parameter to generate a far wall ROI image; and (f) the cIMT delineation module - applies a filter, for example a gaussian filter, on the far wall ROI image to reduce speckle noise and generate a smoothed intensity map, - applies a Sobel filter on the smoothed intensity map in the column direction y to retrieve a y-gradient map of a region of interest, - retrieves continuous paths passing through maximum intensity values defined as a center of adventitia layer, - retrieves all gradient local maxima located above the adventitia center, - selects a pre-defined number of the longest paths and retaining overlapping paths, which are considered as lumen-intima or media-adventitia candidates, the paths located closest to the first side being identified as lumen-intima, and the paths closest to the second side being identified as the media-adventitia, - defines as ROI a section having a pair of paths with the largest overlap.

It is advantageous to provide a system for processing images from brightness mode ultrasound devices that allows an accurate assessment of various cardiovascular health parameters including cIMT, plaque localization and measurement, and arterial stiffness.

It is advantageous to provide a system for assessment of cardiovascular health parameters using brightness mode ultrasound images which is cost efficient.

It is advantageous to provide a system for assessment of cardiovascular health parameters using brightness mode ultrasound images which allows to provide rapid and reliable measurements.

It is advantageous to provide a system for assessment of cardiovascular health parameters using brightness mode ultrasound images which facilitates the practitioner's capture of ultrasound images in a more accurate manner.

Various embodiments have been achieved by providing a system for evaluating cardiovascular health parameters using portable brightness mode ultrasound devices according to the independent claims. Dependent claims set forth advantageous embodiments.

Further advantageous aspects of the various embodiments invention will be apparent from the claims, and from the following detailed description and accompanying Figures.

**Brief Description of the Drawings**

Figure 1 is a schematic block diagram of a B-mode ultrasound image processing system for cardiovascular health assessment based on ultrasound images of a patient's carotid artery according to one embodiment of the invention;

Figure 2a is a flow chart of an image capture process of the system of Figure 1 according to one embodiment, in particular directed to the image capture of a transverse section of the carotid artery;

Figure 2b is a flow chart of an image capture process of the system of Figure 1 according to one embodiment, in particular directed an image capture of the carotid artery along a longitudinal axis;

Figure 2c is a flow chart of a lumen and region of interest (ROI) detection process of the system of Figure 1 according to one embodiment;

Figure 2d is a flow chart of a cIMT and plaque characterization process of the system of Figure 1 according to one embodiment;

Figure 2e is a flow chart of a plaque characterization process according to one embodiment;

Figure 3a is a pictorial illustration of the cardiovascular health parameters that a medical practitioner may assess based on B-mode ultrasound image capture of a patient;

Figure 3b is a screenshot of an example of a measurement output of some health parameters of a patient using a system according to one embodiment;

Figures 4 to 12 illustrate various images and plots illustrating specific examples of processes implemented in the various embodiments:

Figure 4a shows a short axis view of the common carotid artery, where the lumen is located inside the green bounding box;

Figure 4b shows an image displaying the carotid bifurcation inside the red bounding box;

Figure 5a shows an example of a raw B-mode ultrasound image;

Figure 5b shows an example of a binary mask where white pixels correspond to pixels with intensities between the Otsu thresholds T1, T2;

Figure 5c shows a binary mask after removing groups of isolated white pixels;

Figure 6a shows an example of an ultrasound image of a longitudinal view of the carotid.;

Figure 6b shows an example raw image;

Figure 6c shows an example gaussian-filtered image;

Figure 6d shows a standardized intensity profile with local significant maxima flagged with red dots;

Figure 6e shows a standardized intensity profile with lumen candidates flagged with green dots;

Figure 6f: Binary mask where 1's (blue dots) corresponds to lumen axis candidate positions;

Figure 6g: Image representing in different colors 3 different lumen axis paths candidates;

Figure 6h: Raw image with the final lumen axis choice displayed in green;

Figure 7a: Raw image with the lumen displayed in green;

Figure 7b: the resulting image after setting to 0 all pixels above the lumen;

Figure 8c: Binary mask where bright pixels correspond to all pixels whose intensity is above the Otsu threshold T1;

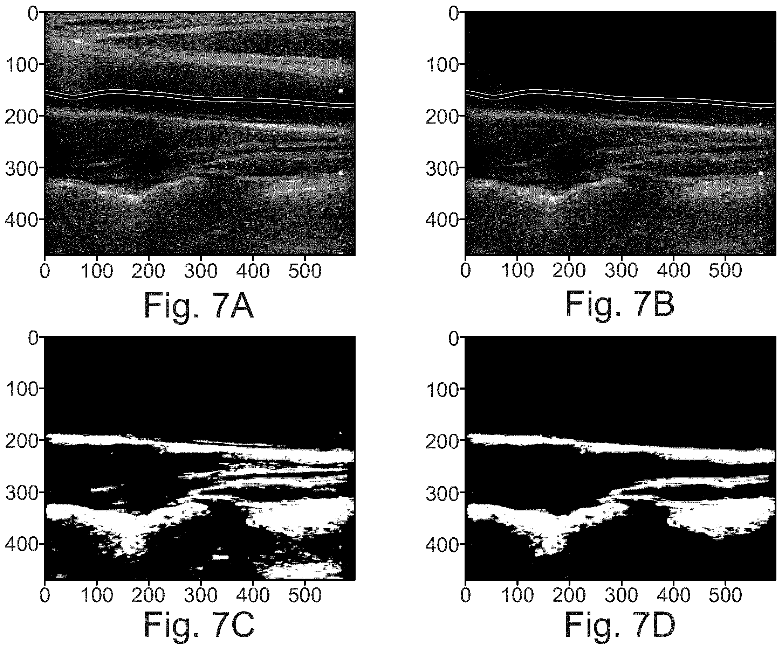

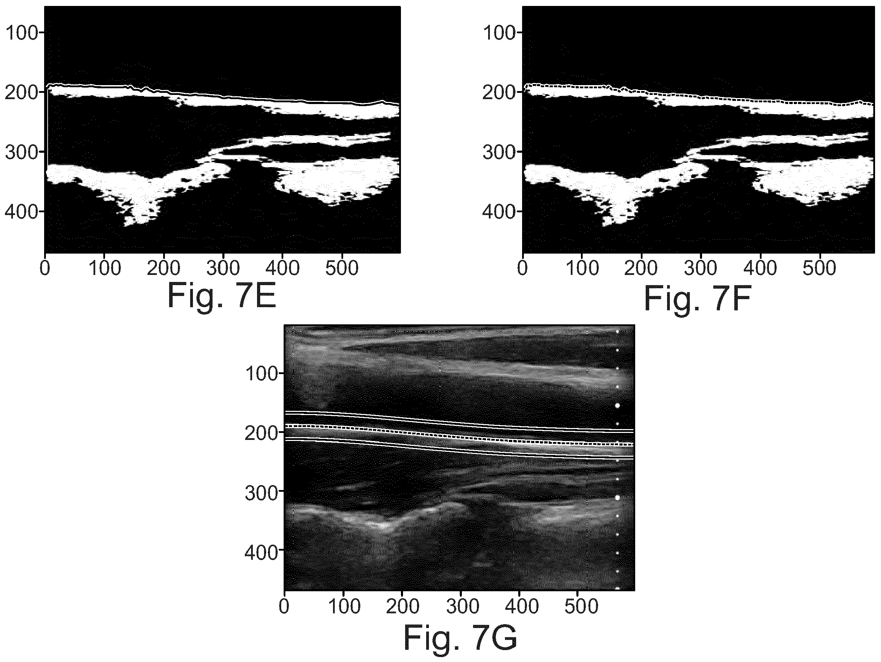

Figure 7d: Binary mask corresponding to the largest bright connected components;

Figure 7e: Binary mask with the first estimate of the far wall displayed in red;

Figure 7f: Binary mask with the final estimate of the far wall displayed in green;

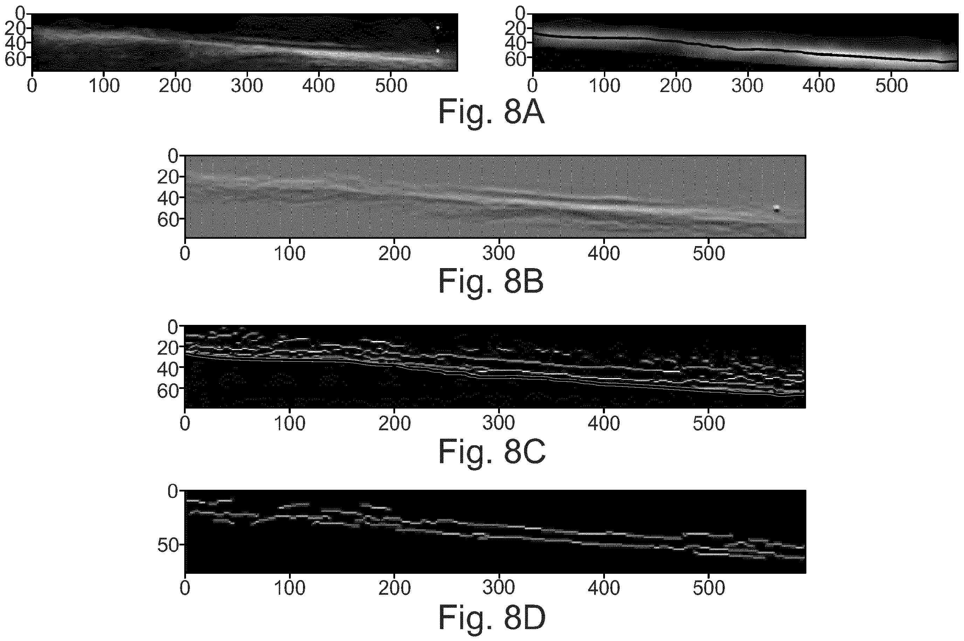

Figure 8a: Far wall ROI raw image (left), and corresponding (Gaussian) smoothed intensity map (left) with the center of the estimated adventitia displayed as a red line;

Figure 8b: y-gradient map of the far wall ROI obtained by applying a Sobel filter on the raw image;

Figure 8c: Binary mask where 1's (white pixels) corresponds to gradient local maxima in the y axis direction, located above the adventitia center. The adventitia center is displayed as a red continuous line;

Figure 8d: Largest 20 connected components that correspond to edges;

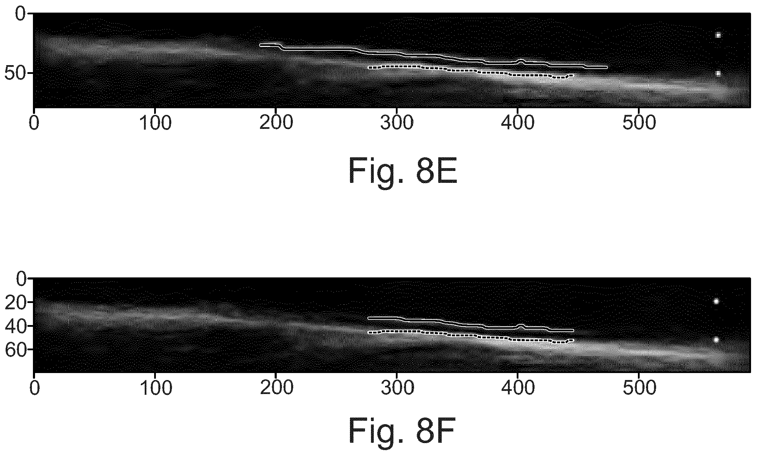

Figure 8e: Final lumen-intima (red) and media-adventitia (yellow) candidates;

Figure 8f: Final lumen-intima (red) and media-adventitia (yellow) delineations;

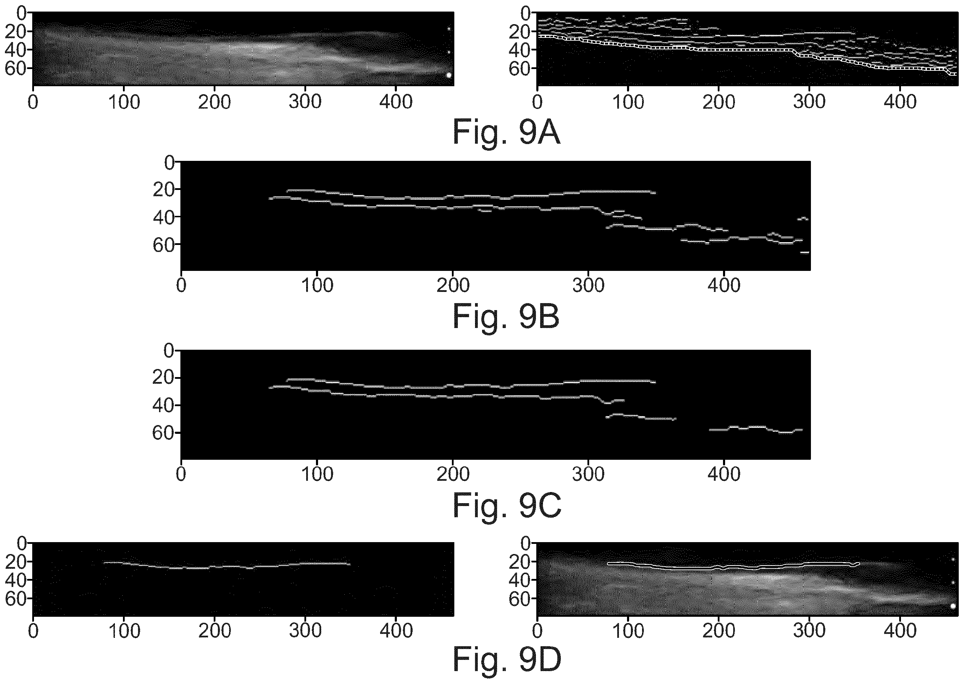

Figure 9a: Raw far wall ROI image (left) and binary mask (right) where 1's (white pixels) corresponds to gradient local maxima in the y axis direction, located above the adventitia center. The adventitia center is displayed as a red continuous line;

Figure 9b: Binary mask where 1's (bright pixels) represents strong edges;

Figure 9c: binary mask where 1's (bright pixels) represents long and strong edges;

Figure 9d: Initial lumen-intima delineation estimate (left), and raw far wall ROI image with the superposed lumen-intima delineation in red (right);

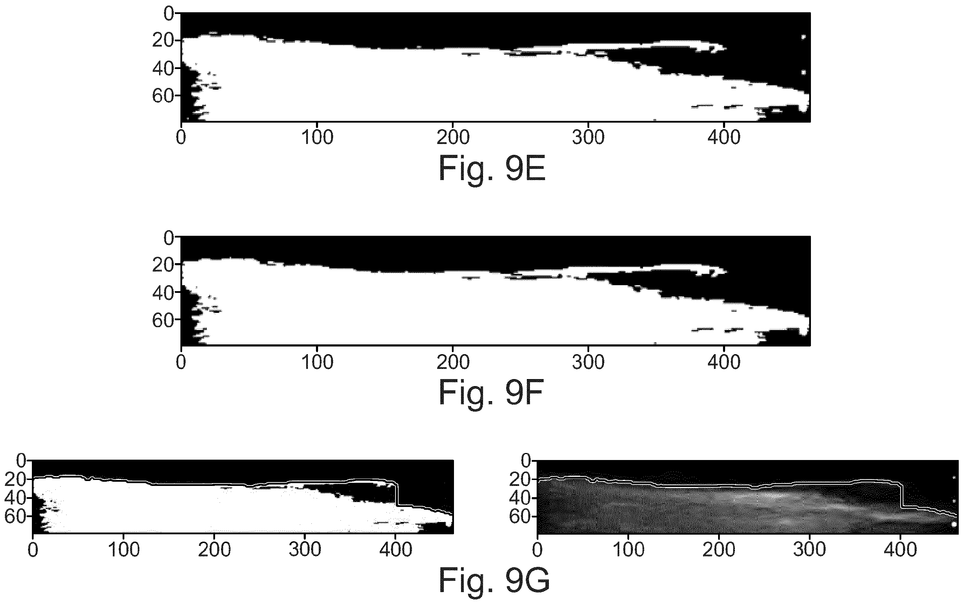

Figure 9e: Binary mask result of thresholding the raw image with the threshold T*;

Figure 9f: Largest connected component of the binary mask computed by thresholding the raw image with T*;

Figure 9g: Largest connected component with the final lumen-intima delineation in red (left), and raw image with the final lumen- intima delineation in red (right);

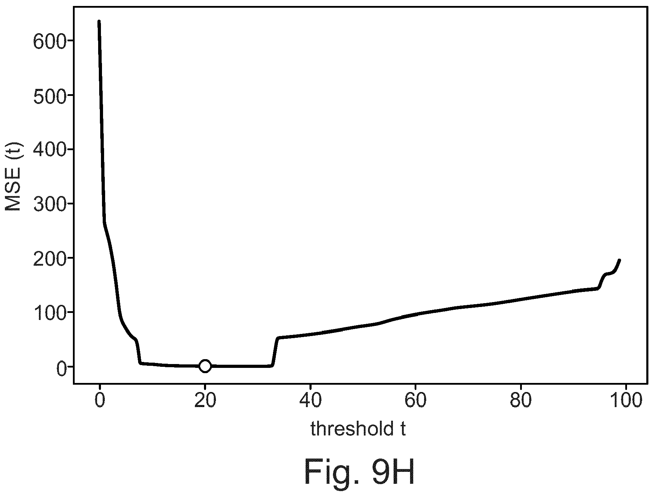

Figure 9h: Graph displaying the MSE against the different values of the threshold t. The MSE is minimized at T*=20;

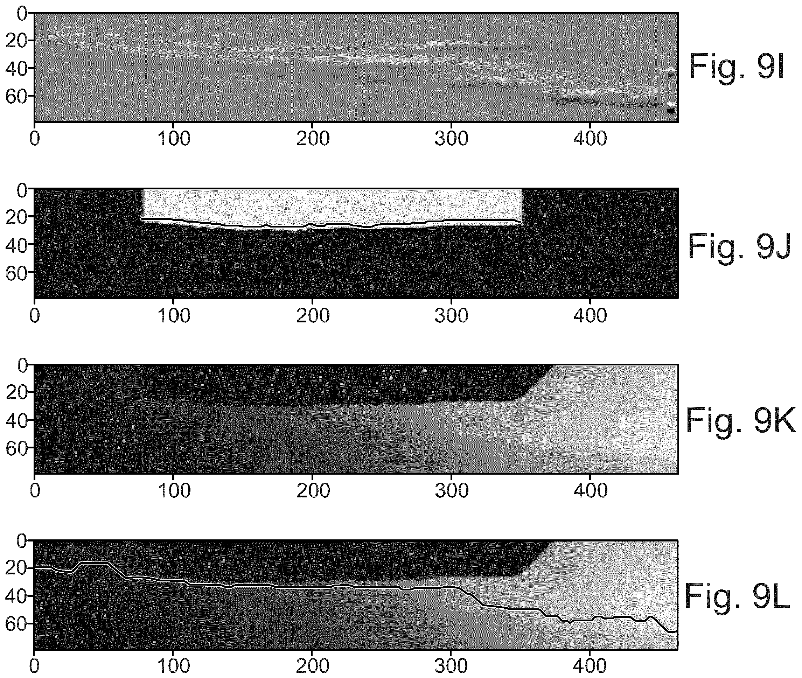

Figure 9i: y gradient map after applying a Sobel filter to the raw far wall ROI image;

Figure 9j: Binary mask where 1's represents the lumen-intima basin. The red line represents the lumen-intima initial estimate;

Figure 9k: Score map C(x,y) ;

Figure 9l: Score map C(x,y) with the optimal path corresponding to the media-adventitia display as a red line;

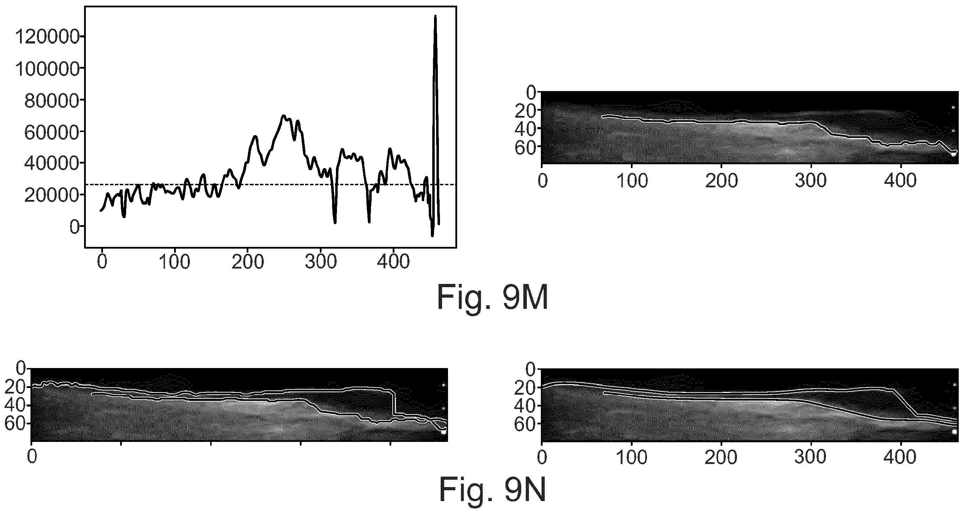

Figure 9m: Left: gradient profile of the media-adventitia pixels. In red a threshold of 0.2 times the global maxima is displayed. Right: raw image with the final media-adventitia delineation displayed in red;

Figure 9n: Left: Final lumen-intima and media-adventitia delineations for plaque. Right: Final smoothed lumen-intima and media- adventitia delineations for plaque;

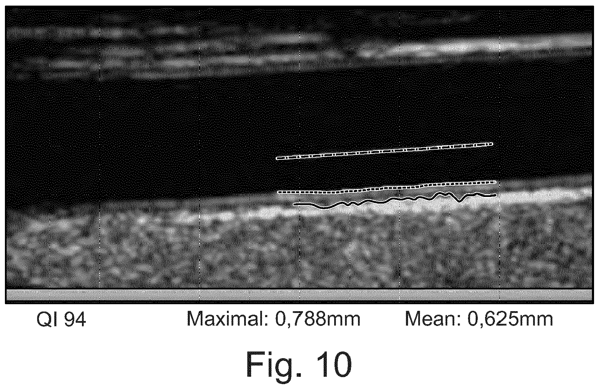

Figure 10: Image taken from Philipps QLab software, the QI=94 means that 94% of the points in the desired area were successfully delineated;

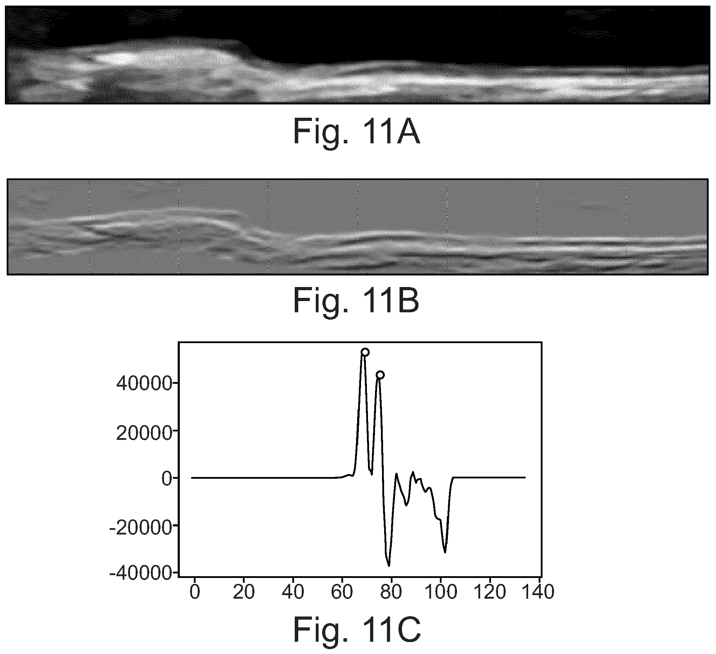

Figure 11a: Far wall ROI image;

Figure 11b: Gradient map of the far wall ROI image;

Figure 11 c: Gradient profile of a fixed column, with lumen-intima and media-adventitia points marked in red;

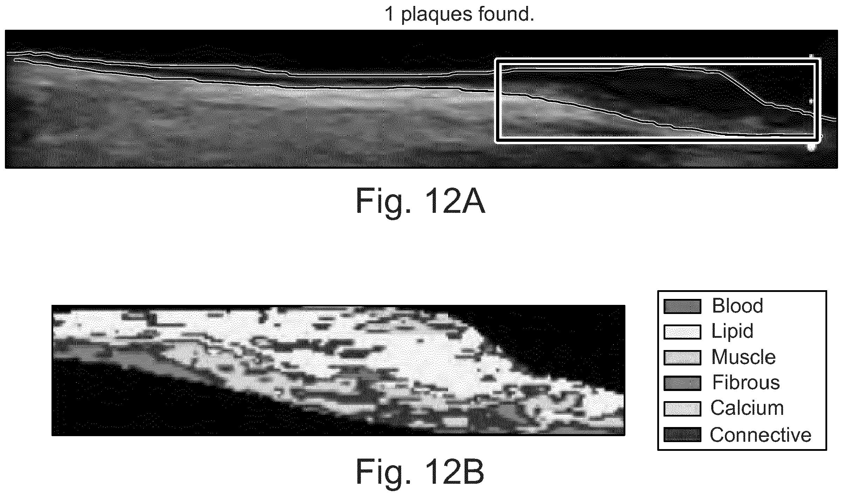

Figure 12a: Far wall ROI raw image with the cIMT delineation in dark green and the plaque region located inside the light green bounding box;

Figure 12b: Plaque composition according to pixel grayscale intensities, and

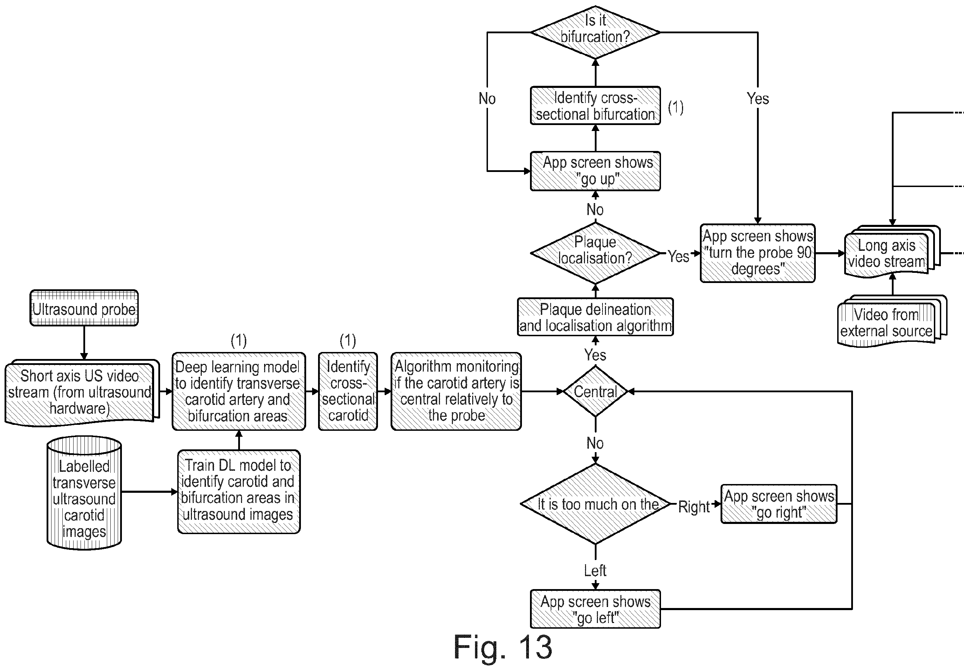

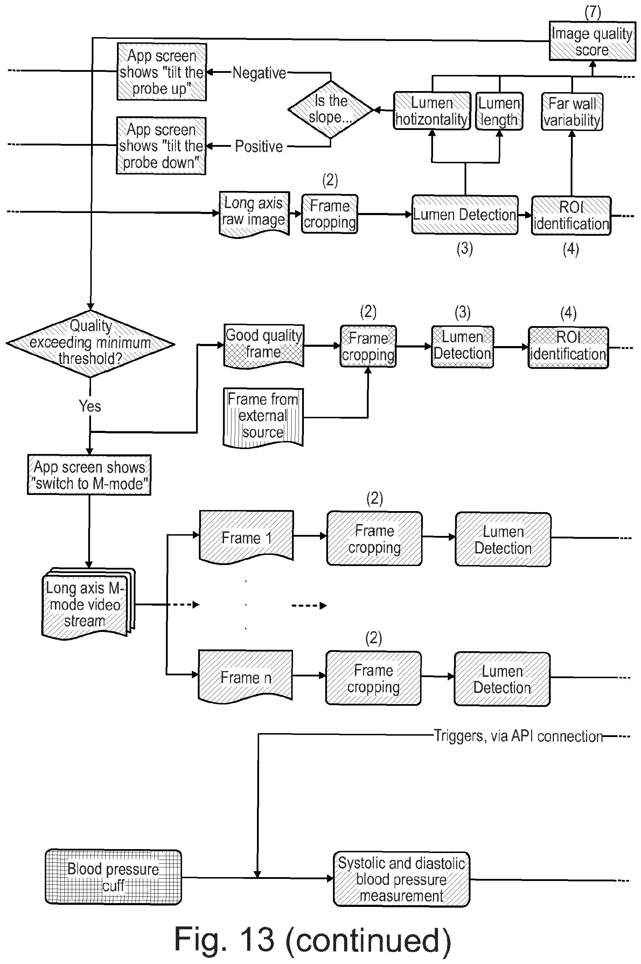

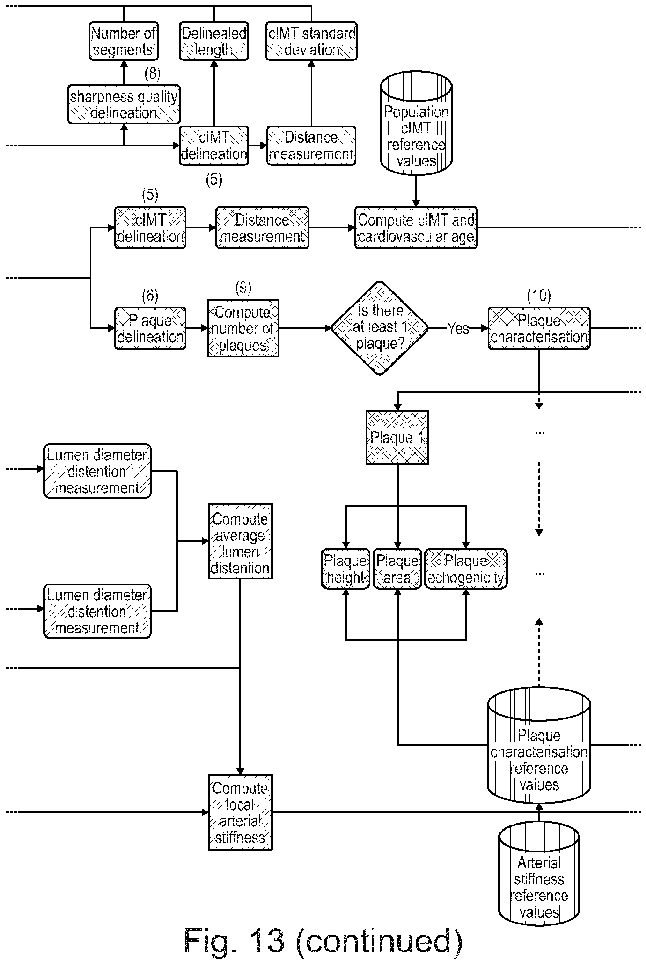

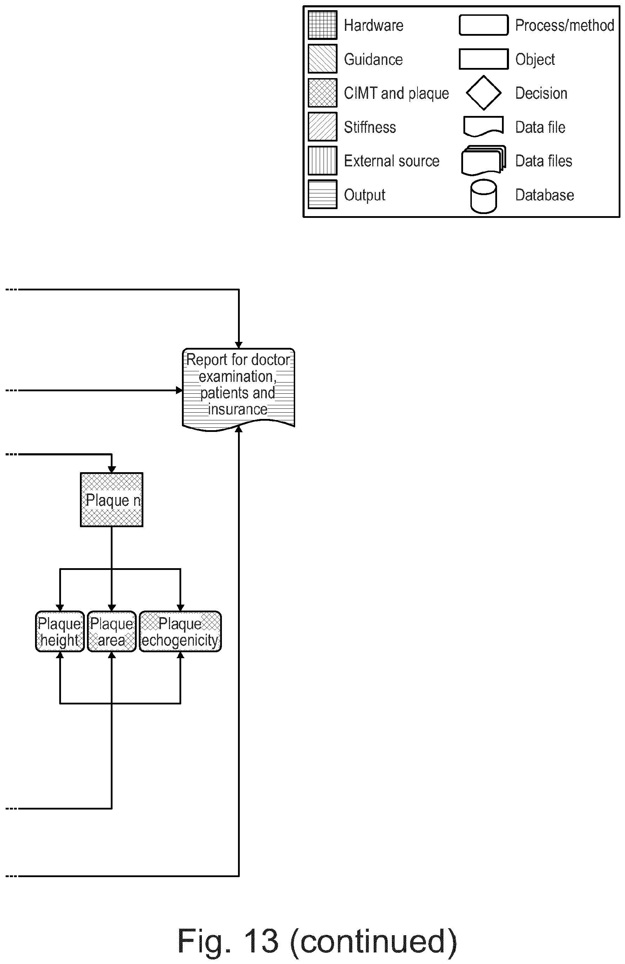

Fig. 13 shows a flow chart corresponding to another embodiment of an ultrasound image processing system for cardiovascular health assessment based on ultrasound images of a patient's carotid artery according to one embodiment of the invention.

The drawings are not necessarily to scale. In general, like numbers refer to like parts or steps throughout the drawings.

**Detailed Descriptions of Some Embodiments**

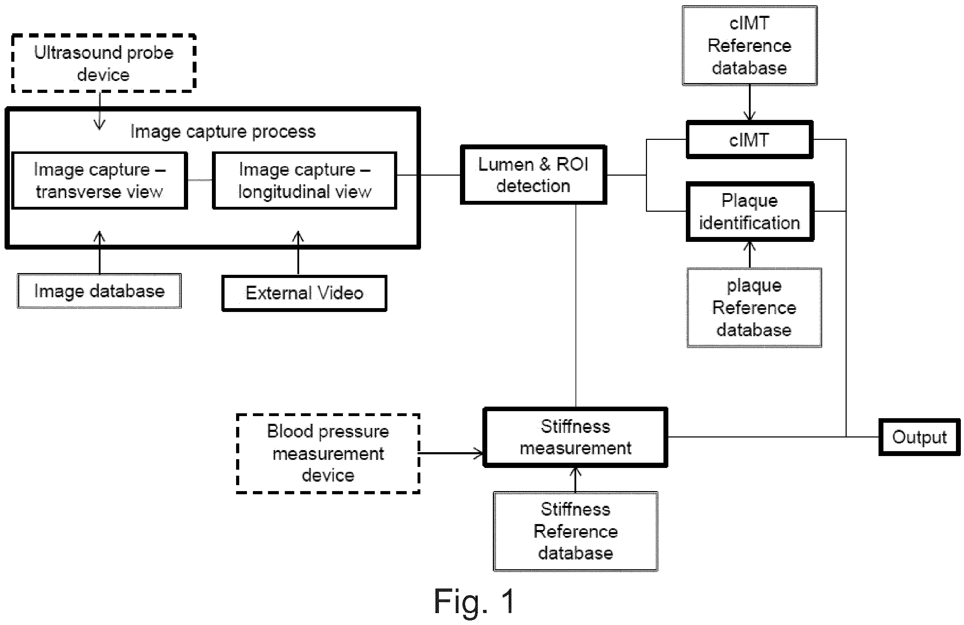

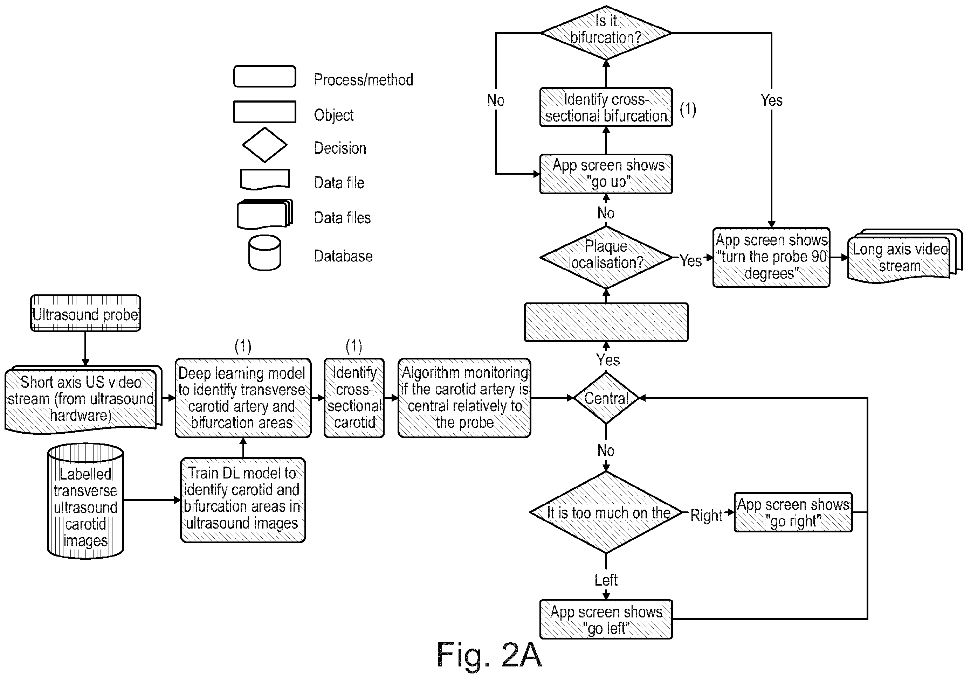

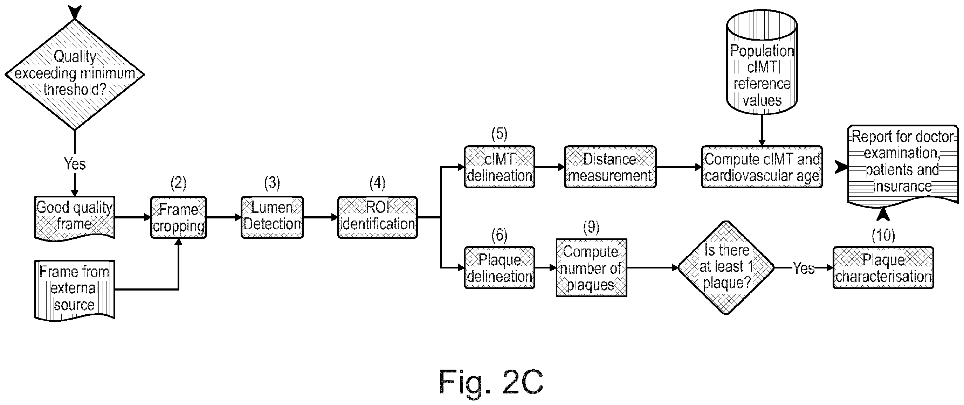

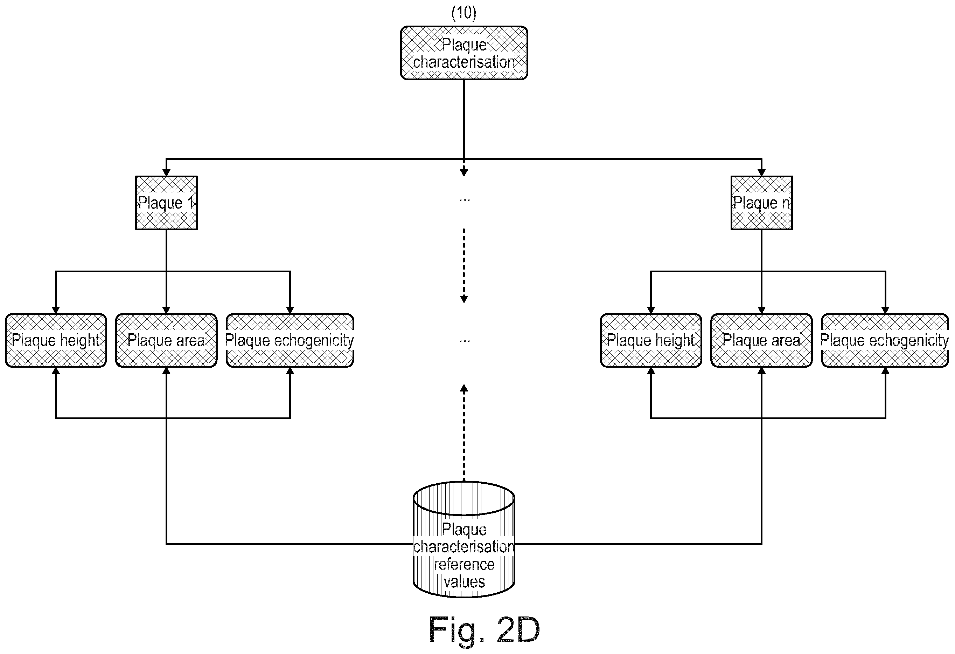

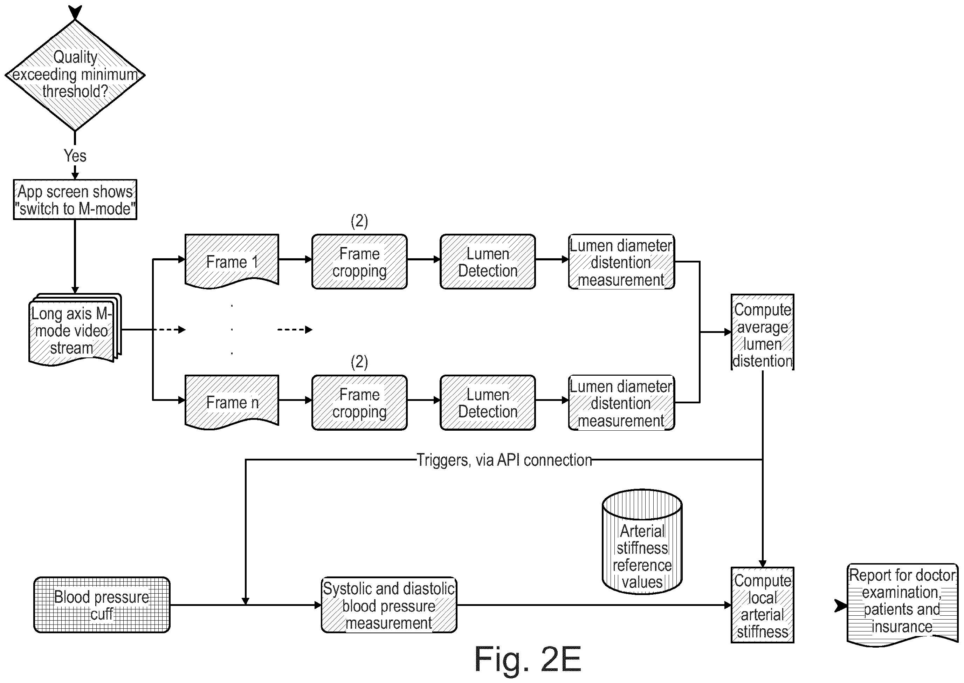

Referring to the Figures, starting in particular with Figure 1 and Figures 2a to 2e, a B-mode ultrasound image processing system for measurement of cardiovascular parameters is schematically illustrated. The processing system according to some embodiments comprises a computing system with program modules installed in the computing system. The program modules are configured to process data received from an ultrasound probe device, in particular a portable ultrasound probe device that may be a commercial off the shelf portable ultrasound device used by medical practitioners for brightness mode (B-mode) ultrasound image capture of organs in a patient's body, including of a patient's carotid artery. The data transferred by the ultrasound probe device includes in particular a video stream of the ultrasound images picked up by the ultrasound probe device during medical investigation of a patient's internal organs by the medical practitioner. Other measurement devices may be connected to the computing system, for example a blood pressure measurement device that may in particular be used to supply data on the patient's blood pressure that may be used *inter alia* for arterial stiffness measurement as will be described in more detail hereafter.

In one embodiment, the computing system includes a memory that may store various databases. The computing system may be connected to a communications network and configured to access various databases on external servers or on the cloud. The databases may comprise an image database of still and/or moving images that may be used as a reference database for a comparison with images captured by the medical practitioner or that may be used for training an artificial intelligence program for improving the processing of image data for more accurate measurement of cardio vascular parameters according to this invention. Artificial intelligence programs are per se well known and need not be further described herein.

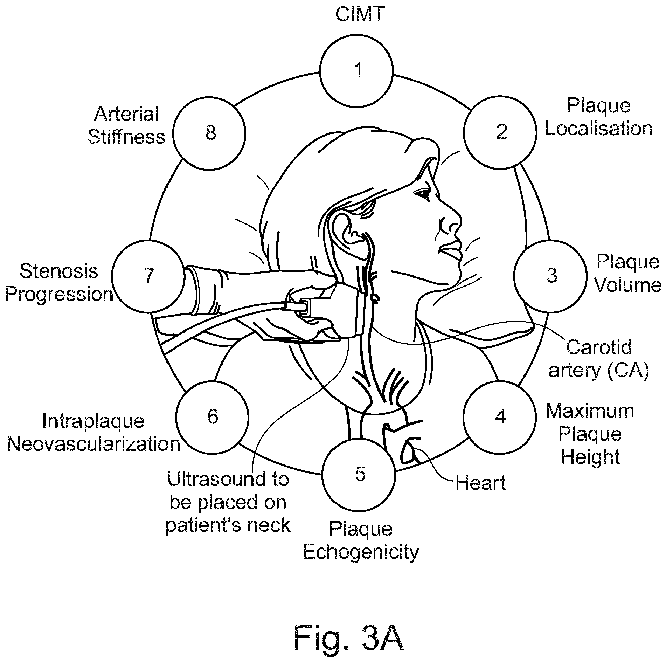

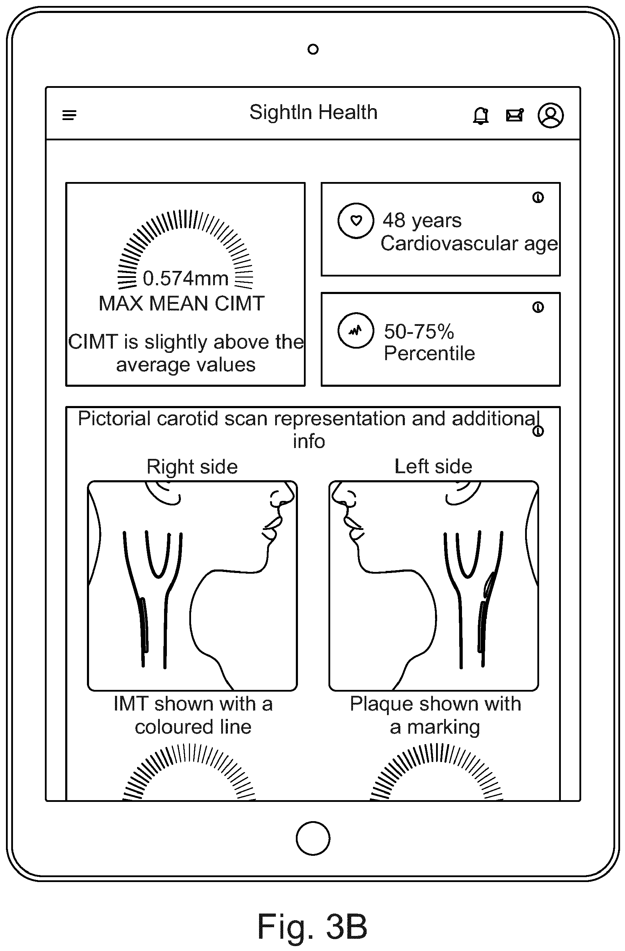

In one embodiment, the computing system may include an output device that may include for example a computer, smartphone, or smartpad with a graphical user interface to display cardiovascular health measurement parameters as illustrated for example in Figure 3b. The displayed information may be adapted for consultation of measurement results by the medical practitioner, or by the patient. The output device may be a separate device or may be integrated in a computer connected to the portable ultrasound device.

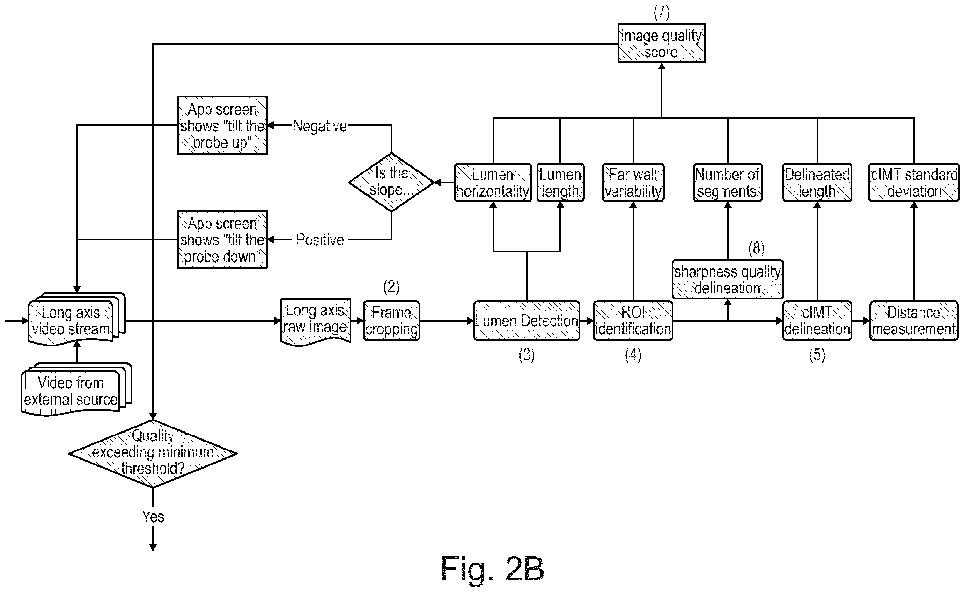

In some embodiments, various program modules installed in the computing system execute various processes for cardiovascular health parameter measurement. The program modules can include an image capture module that receives and processes incoming video streams from the ultrasound probe device or an external video source. The image capture module may be configured to display the captured ultrasound image on a screen of the computing system visible to a medical practitioner during the image capture process, whereby the image capture process module may provide feed-back to guide the medical practitioner. In particular, the image capture process may be configured to guide the medical practitioner to scan the carotid in the short axis until the carotid bifurcation is reached and then to indicate when the ultrasound probe may be reoriented to start scanning the carotid in the long axis. This allows for example to measure atherosclerotic plaque and cIMT. An example of a process of transverse carotid and bifurcation identification is described in more detail hereafter and illustrated in Figures 2a and 2b.

According to some embodiments, detailed examples of lumen and ROI detection are provided in the detailed examples hereafter. The process flow chart of the image capture process module illustrated as an example in Figures 2a and 2b also illustrates a process of lumen detection and region of interest (ROI) determination included in the image capture processing.

According to some embodiments, one feature is the manner in which the lumen and region of interest are identified and extracted for further processing to determine the cIMT, identify and characterize plaques, and measure arterial wall stiffness in conjunction with a blood pressure measurement device.

According to some embodiments, the program modules may function generally in the following manner.

A raw image taken substantially along a plane parallel to the carotid longitudinal axis, showing a longitudinal cross-section of the carotid artery captured by an ultrasound probe device, is received by the computing system and processed by the program modules. The image may also be received from an image database, or an external video.

The program modules process the image for measurement of parameters relevant to the assessment of cardiovascular health.

In some embodiments, the overall process of the system may include one, selected ones, or all of the following steps:

1. Transverse Carotid and Bifurcation Identification
2. Frame Cropping
3. Lumen Delineation
4. Far Wall ROI Segmentation
5. cIMT Delineation
6. Plaque Delineation
7. Image Quality Delineation
8. Sharpness Quality Delineation
9. Plaque Identification
10. Plaque Characterization
11. Wall Tracker
12. Waveform Processor

**1. Example of Transverse Carotid and Bifurcation Identification**

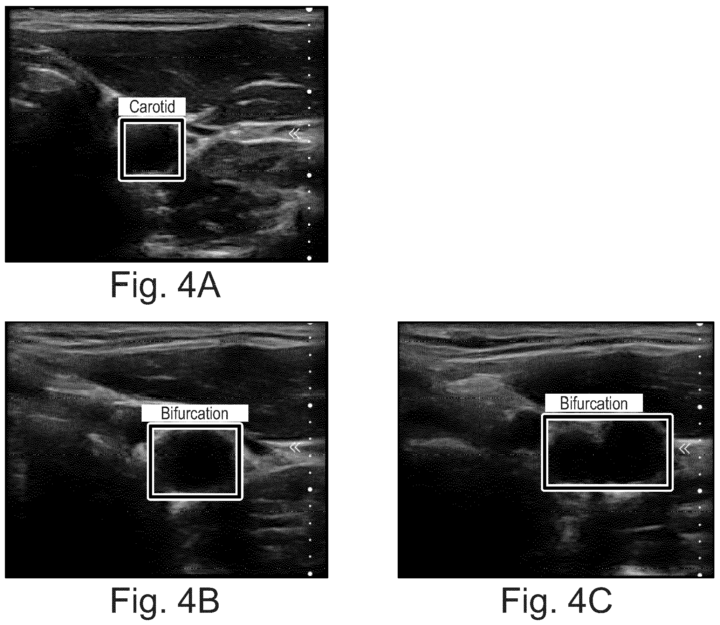

The practitioner is guided by the system to scan with the ultrasound probe device the carotid in the short axis (Figure 4a) until the carotid bifurcation is reached (Figures 4b and 4c) and then is guided to turn the ultrasound probe device about 90° to start scanning in the long axis for measurement of atherosclerotic plaque and cIMT. In order to follow this process, the practitioner needs to be able to identify both the carotid lumen and bifurcation in the short axis view for which assistance is provided by the system that identifies and marks the carotid and the bifurcation.

Although there are some prior studies [1], [2] on using Deep Learning model architectures to locate the carotid lumen structure in the short axis, such procedures have not been suggested to locate the bifurcation region as proposed in the present invention.

**Example of Identifying a Carotid Lumen**

A Convolutional Neural Network (CNN) such as a YOLOv5 object identification Deep Learning model may be trained to identify the transverse view of the common carotid artery. More specifically, bounding boxes for the carotid artery in the cross-sectional view are labelled, and then the Deep Learning algorithm can be trained to learn to identify such structures. In Figure 4a one may observe a short axis view of the carotid artery, with the corresponding bounding box. [see Figure 4(a): Short axis view of the common carotid artery, The lumen is located inside the green bounding box.]

**Example of Identifying a Carotid Lumen Bifurcation**

The method assumes as input a raw image with the carotid lumen bounding box position(s). When the carotid starts to bifurcate, the substantially circular lumen first gets deformed into an oval shape, and right after, the two lumens corresponding to the internal (ICA) and external (ECA) carotid arteries start to be distinguished. Thus, the carotid bifurcation is identified by the program module if one of the two following conditions is satisfied:

(i) The ratio of the length of the sides of the bounding box is above a certain fixed threshold, [see Figure 4b: Image displaying the carotid bifurcation inside the red bounding box. In this image the bifurcation is identified because the bounding box of the carotid has a larger length than height.]
(ii) There are two bounding boxes for the carotid lumen that are overlapping. [see Figure 4b: Image displaying the carotid bifurcation inside the red bounding box. In this image the bifurcation is identified because there are two overlapping bounding boxes for the carotid lumen (corresponding to the ICA and the ECA.]

**2. Example of Frame Cropping**

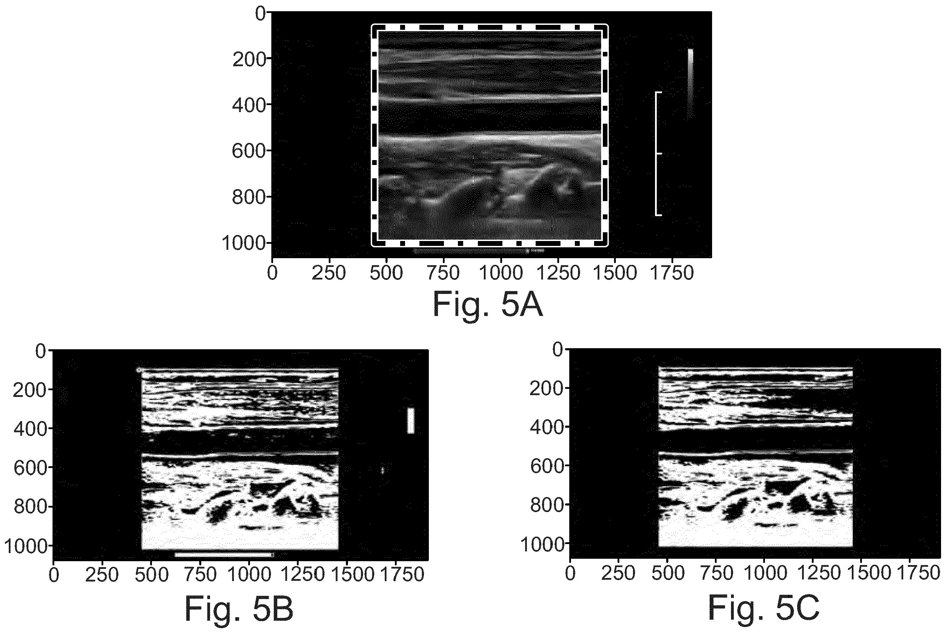

Before any analysis, B-mode ultrasound images (Figure 5a) need to be cropped to get rid of the background/metadata information that is surrounding the image frame.

[see Figure 5a: Raw B-mode ultrasound image.]

One example of an algorithm for frame cropping may be based on a known cropping algorithm, for example as described in [3], with substantial modification for use in some embodiments.

In one embodiment, the frame cropping algorithm assumes the image has 3 classes of pixels:

- Dark pixels, which correspond to the image background or hypoechogenic tissues like blood
- Medium bright pixels, which correspond to echogenic tissues
- Very bright pixels, which correspond to letters, graphical markings, or wall interfaces (very echogenic tissue)

According to some embodiments, the method may be implemented in the following steps:

- The program module applies compute two thresholds, a first threshold T1, and a second threshold T2, that separate these three classes of pixels, using for example a method as described in [4] named herein as the Otsu method;
- The program module retrieves a binary mask (Figure 5b), where 1's (bright pixels) corresponds to all pixels whose intensity values lie between T1 and T2. [see Figure 5b: Binary mask where white pixels correspond to pixels with intensities between the Otsu thresholds T1, T2];
- Groups of connected bright pixels with less pixels that a certain threshold are set to 0. Groups of connected bright with at least one pixel lying in the top edge of the image are also removed. Figure 5c displays the binary mask after removing isolated pixels. [see Figure 5c: Binary mask after removing groups of isolated white pixels.]

In one embodiment, the final cropped image is defined as the smallest rectangular region enclosing all remaining bright pixels. The rectangular dotted line in Figure 5a represents the region of the final cropped image corresponding to the raw frame.

### 3. Example of Lumen Delineation

In carotid ultrasound images, interesting biomarkers such as cIMT, or atherosclerotic plaque are usually measured at the far wall, which is the artery wall located adjacent the lumen, on the far side of the lumen with respect to the ultrasound probe device that captures the image.

For ease of reference, it may be noted that in all of the illustrated brightness mode ultrasound images in the appended Figures, the tissue closest to the ultrasound probe is at the top of the image and the tissue furthest from the ultrasound probe device is at the bottom of the image.

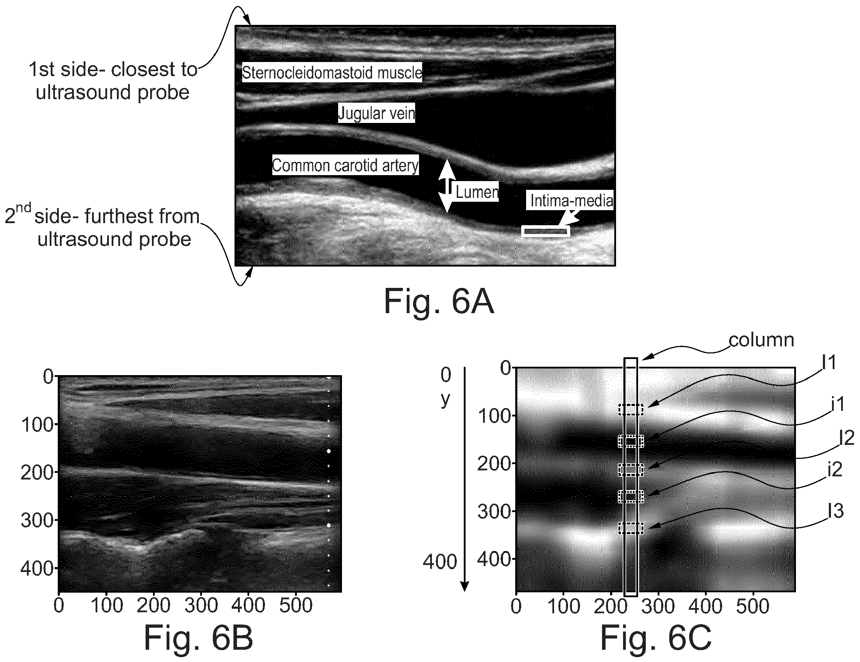

The identification and localization of the lumen structure is as such a common task in ultrasound image analysis for carotid artery analysis [see Figure 6a: Ultrasound image of the longitudinal view of the carotid. Source: [5] Robust common carotid artery lumen detection in B-mode ultrasound images using local phase symmetry]. The general idea of locating the lumen as the minima of a smoothed version of a cropped image is also known from [5], [6], [7].

In prior known methods, the correct location of the lumen is however often difficult or prone to error and moreover does not identify an optimal region of interest for the subsequent analysis of cIMT.

According to one embodiment, therefore, the system enables accurate automated identification and delineation of the lumen axis and identification of a region of interest (ROI) in the far wall for an accurate and reliable subsequent automated assessment of cIMT and optionally other cardiovascular health parameters such as plaque identification and arterial stiffness analysis.

In one embodiment, the algorithm of the program module for lumen delineation is to configured to locate the lumen axis and to allow for a subsequent selection of a high-quality region of interest. The algorithm flow may thus include: The cropped image is first smoothed using a smoothing filter, for example a 2D Gaussian image smoothing filter. [see Figure 6b: Raw image and Figure 6c gaussian-filtered image.]

The resulting pixel intensity values may advantageously be standardized by scaling the values within a reference frame, for example scaled to values lying between 0 to 1.

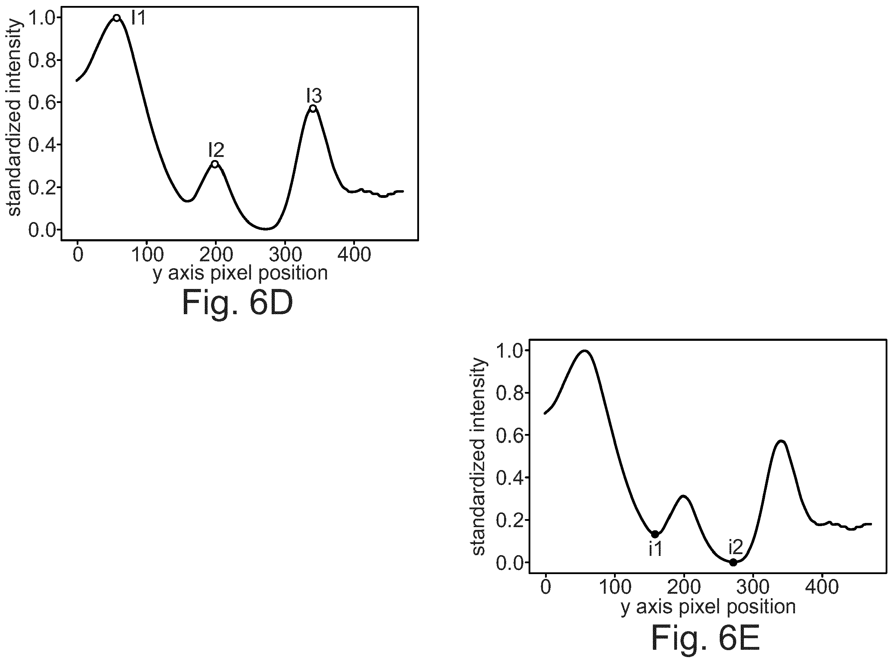

The image may be divided in a plurality of columns for processing column by column, so for each column pixel brightness intensity values lie for example between 0 and 1. A column may comprise a plurality of pixels in width, for example in a range between 3 to 100 pixels, depending on the chosen resolution, whereby the pixel intensity of a row of pixels across the column width is computed for example as a mean value. Pixel brightness intensity values for a given column of the smoothed image are illustrated for example in the plots of Figures 6d and 6e. In these plots, the $y$ axis is the vertical axis and depicts the pixel intensity going from the top (positioned closer to the ultrasound probe device) to the bottom (positioned further from the ultrasound probe device) of the cropped and smoothed image.

Then, and in one embodiment, for each column, the lumen axis candidates may be identified by the program module as follows:

The significant local intensity maxima I1, I2, I3 are identified (e.g., by computing the derivatives of the plot to find the peaks) and retaining maxima that exceed a fixed intensity value threshold, for example a fixed intensity threshold having a value between 0.1 and 0.3, for example set at 0.2. Figure 6d represents the intensity profile of a10th column of Figure 6c by way of example. Three local maxima are identified and all of them are above a chosen fixed threshold of 0.2. [see Figure 6d: Standardized intensity profile with local significant maxima flagged with red dots.]

After identification of the significant local intensity maxima I1, I2, I3, between rows defined by the first and the last significant intensity maxima I1, I3, two smallest significant local intensity minima i1, i2 [see Figure 6e: Standardized intensity profile with lumen candidates flagged with green dots.], are identified (e.g., by computing the derivatives of the plot to find the troughs). The computed two smallest significant minima i1, i2, between the first and the last significant intensity maxima, are retained as lumen candidates. The choice of two candidates instead of one is made to avoid missing the actual lumen in images where the jugular vein, or other dark structures are also present.

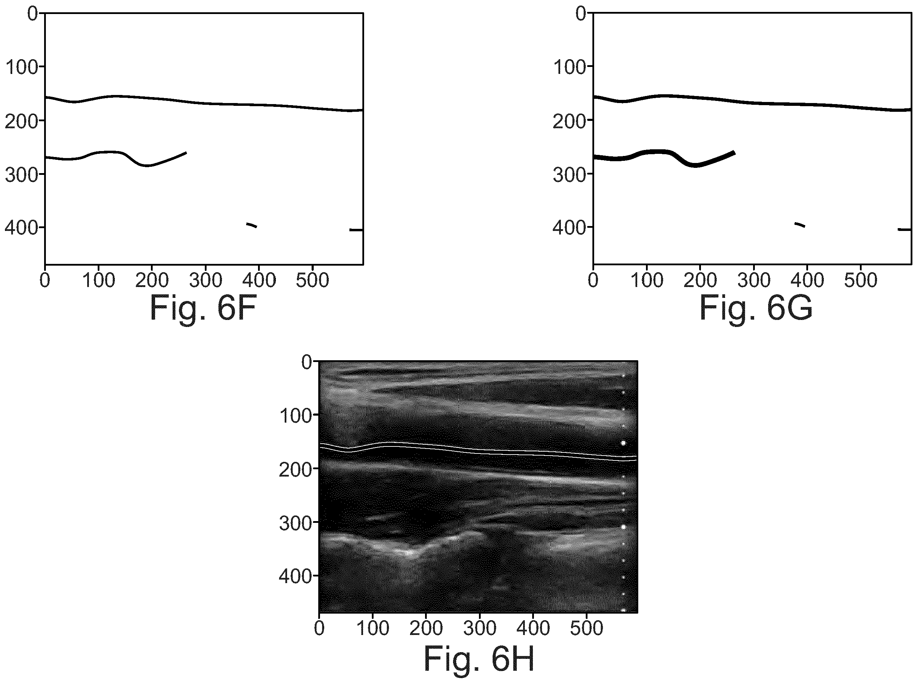

- A binary mask where 1's corresponds to the lumen candidate positions found in the previous step is generated. [see Figure 6f: Binary mask where 1's (blue dots) corresponds to lumen axis candidate positions]
- The program module sets to 0 all isolated 1's pixels in the binary mask and then groups all bright pixels into separate lumen axis candidates' paths.
- From left to right, the program module selects the first column $c\_i1$ with any bright pixels, and initializes lumen paths as $s\_1 = [p\_i1,j1],...,s\_n = [p\_in,jn]$, where $p\_i,j$ are the coordinates of the bright pixels in column $c\_i$
- For each subsequent column $ik$:
- For each bright pixel $p\_ik,jl$, the y axis distance between $p\_ik,jl$ and all the end-points $s\_1[-1],...s\_n[-1]$ is computed. If the minimum distance of all pairs is below a certain threshold, the program module appends $p\_ik,jl$ to the path $s* = argmin(distance(p\_ik,jl, s\_m))$ for m=1,...n.
- If such vertical distance is above a certain fixed threshold, this lumen candidate is considered as the beginning of a new path $s\_n+1 = [p\_ik,jl]$. [see Figure 6g: Image representing in different colors 3 different lumen axis paths candidates.]

According to one embodiment, all paths whose length is above a chosen proportion *x* times the longest path length are retrieved, and the lumen is identified as the path furthest from the ultrasound probe device (bottom-most path in the illustrated images). By selecting a conservative threshold, for example of *x* above 0.8, for example *x*=0.9, the final lumen candidate is the green line represented in Figure 6h. [see Figure 6h: Raw image with the final lumen axis choice displayed in green.]

### 4. Example of Far Wall ROI Segmentation

Both the cIMT and atherosclerotic plaques are measured at the far wall region of the common carotid artery, which is what is defined herein as the selected region of interest (ROI). Thus segmentation of the far wall is a step needed before delineating the intima-media and media-adventitia interfaces. As the far wall is located below the lumen, the selected far wall identification method requires the lumen axis position as an input parameter.

Although the idea of defining the ROI as an envelope around the media-adventitia interface is *per se* known from [8], the optimum selection of the ROI based on the lumen delineation as described above and the longest connected path selection as described below according to an advantageous embodiment was not previously known and allows to select an optimal ROI for accurate and reliable cIMT calculation.

According to one embodiment, the flow of the ROI segmentation algorithm may be as follows:

The intensity of pixels above the lumen is set to 0 [see Figure 7a: Raw image with the lumen displayed in green and Figure 7b: the resulting image after setting to 0 all pixels above the lumen.]
A threshold T1 using an Otsu thresholding method is computed to separate pixels into two classes according to their brightness:

- Dark pixels corresponding to the lumen, all pixels above it, and other hypoechoic tissues
- Bright pixels corresponding to the wall interfaces below the lumen and other hyperechoic tissues.

A binary mask setting to 1's all pixels above the Otsu threshold T1 is computed [see Figure 8c: Binary mask where bright pixels correspond to all pixels whose intensity is above the Otsu threshold T1].
A new binary mask with all large connected components is retrieved. A connected component is defined as "large" if the number of connected pixels is above a selected proportion of the number of connected pixels of the largest connected component [see Figure 7d: Binary mask corresponding to the largest bright connected components]. The selected proportion is preferably in a range of 0.5 to 1, preferably 0.7 to 1.

For each column, the far wall vertical position y is identified as the first bright pixel (from top to bottom in the image of the Figure 7e) of the binary mask. [see Figure 7e: Binary mask with the first estimate of the far wall displayed in red.] Once the estimated the vertical *y* coordinates array for the far wall are identified, the final estimate is defined as the largest sub-segment not containing any NaNs or jumps exceeding a fixed threshold. In Figure 7f one may observe how the green line corresponding to the final far wall estimate is not containing the initial jump of the red line in Figure 7e [see Figure 7f: Binary mask with the final estimate of the far wall displayed in green.].

Finally, the far wall array may be smoothed with smoothing filter, for example a Savitzky-Golay filter, and the upper and lower bounds of the far wall ROI are defined with a predefined height parameter falling within a preferred range. In Figure 7f the green line corresponds to the smoothed far wall and the orange lines correspond to the lower and upper bounds of the ROI.

## 5. Example of cIMT Delineation

The cIMT is defined as the distance between the lumen-intima interface and the media- adventitia interface, and it is typically measured at the far wall of the common carotid artery.

Some aspects relating to locating the adventitia as the brightest line and then restricting the lumen- intima and media-adventitia search above that line are discussed in [10], and some aspects of lumen-intima and media-adventitia as parallel continuous lines along maximum gradient paths are described in [11].

However, according to some embodiments described and disclosed herein, the cIMT delineation algorithm of the program modules performs the cIMT delineation in the provided far wall region of interest. In one embodiment, the algorithm flow is as follows:

The cIMT delineation algorithm applies a filter, for example a gaussian filter, on the far wall ROI image to reduce speckle noise. We refer to the resulting image as the smoothed intensity map I.

The center of the adventitia layer is retrieved as the continuous path passing through maximum intensity values. In other words, the adventitia layer is retrieved as the path that maximizes the score map defined as $C(x, y) = \max(C(x-1,y-1),C(x-1,y),C(x-1,y+1)) + I(x,y)$, where $I(x,y)$ are the values of the smoothed intensity map. The score map and the path optimization may be computed with a Dynamical Programming (DP) approach so that the computational cost is minimal. [see Figure 8a: Far wall ROI raw image (left), and corresponding (Gaussian) smoothed intensity map (left) with the center of the estimated adventitia displayed as a red line.]

A Sobel filter is applied in the vertical direction y to retrieve the *y*-gradient map of the region of interest, [see Figure 8b: *y*-gradient map of the far wall ROI obtained by applying a Sobel filter on the raw image.]

In one embodiment, the program module retrieves all the gradient local maxima located above the adventitia center. Indeed, one is looking for the media-adventitia, and lumen-intima edges, so one should discard all edges after the adventitia. [see Figure 8c: Binary mask where 1's (white pixels) corresponds to gradient local maxima in the *y* axis direction, located above the adventitia center. The adventitia center is displayed as a red continuous line.]

The program module selects a pre-defined number of the largest connected components, for example the 20 largest, which are considered as possible lumen-intima or media-adventitia candidates. The number 20 is arbitrary but for example may be based on a value selected by a programmer after visual inspection and validation of several images, [see Figure 8d: Largest 20 connected components that correspond to edges.]

The program module retrieves the lumen-intima and media-adventitia paths as follows:

If the two largest connected components $c_1$, $c_2$ have a significant overlap (above a fixed threshold, in our case 0.3 times the largest connected component length) in the *y* axis, they are selected as final candidates. Otherwise, the overlaps between $c_1,c_2$ are computed and all the other largest connected components $c_3,...,c_{20}$. The program module then selects as final candidates the pair with the largest overlap. From the final pair of candidates, the uppermost path is identified as lumen-intima, and the lowermost path is identified as the media-adventitia

If the two largest connected components $c_1$, $c_2$ have a significant overlap (above a fixed threshold, in our case 0.3 times the largest connected component length) in the *y* axis, they are selected as final candidates. Otherwise, the overlaps between $c_1,c_2$ are computed and all the other largest connected components $c_3,...,c_{20}$. The program module then selects as final candidates the pair with the largest overlap [see Figure 8e: Final lumen-intima (red) and media-adventitia (yellow) candidates]

The final lumen-intima and media-adventitia delineations can be seen in Figure 8f, and are given as the delineations in the columns where both lumen-intima and media-adventitia are identified [see Figure 8f: Final lumen-intima (red) and media-adventitia (yellow) delineations].

## 6. Example of Plaque Delineation

According to some embodiments, the aim of this method is to provide a delineation throughout the whole ultrasound image so that if there is any plaque it can be identified. In contrast with the cIMT delineation, where we are just interested in delineating a small region where we can accurately measure the cIMT, delineation towards plaque identification is run throughout the whole image.

The idea of locating the media-adventitia as a path that maximizes the gradient was already introduced in [12]. Another fully-automatic plaque delineation can be found in [13].

However, and according to some embodiments, the algorithm flow can be divided into 3 stages:

**Example of Lumen-Intima Initial Delineation**

According to one embodiment, the flow of this first stage is the following:

Follow steps 1-4 of the cIMT method to obtain all the gradient local maxima located above the adventitia center [see Figure 9a: Raw far wall ROI image (left) and binary mask (right) where 1's (white pixels) corresponds to gradient local maxima in the y axis direction, located above the adventitia center. The adventitia center is displayed as a red continuous line.].

Connected components or paths where all pixels have gradient values below a certain fixed threshold are considered as weak edges and set to 0 by the program module [see Figure 9b: Binary mask where 1's (bright pixels) represents strong edges].

The program module further sets to 0 all edges whose length is below a certain fixed threshold [see Figure 9c: binary mask where 1's (bright pixels) represents long and strong edges].

By definition, the lumen-intima edge is right after the lumen, and above the media-adventitia, so we should expect that there are no edges above it, but there are edges below it. Thus, the program module selects as initial lumen-intima candidates those edges containing:

• a minimum fixed % of pixels don't have any other bright pixel above.
• a minimum fixed % of pixels that have other edge pixels below.

[see Figure 9d: Initial lumen-intima delineation estimate (left), and raw far wall ROI image with the superposed lumen-intima delineation in red (right).]

**Example of Lumen-Intima Final Delineation**

According to one embodiment, and given the lumen-intima initial estimate computed in the steps described in the first stage above, the flow of the second stage is the following:

Given an optimal threshold T* (detailed computation shown below), the program module retrieves a binary mask with 1's corresponding to all pixels whose intensity value is above T* [see Figure 9e: Binary mask result of thresholding the raw image with the threshold T*].

The program module retrieves the largest connected component of the mask computed in the preceding step [see Figure 9f: Largest connected component of the binary mask computed by thresholding the raw image with T*.].

The final lumen-intima delineation is computed as the array of uppermost positions of the largest connected component retrieved in the preceding step [see Figure 9g: Largest connected component with the final lumen-intima delineation in red (left), and raw image with the final lumen- intima delineation in red (right).].

The computation of the optimal threshold T* used in the above three steps is performed as follows:

For each integer threshold candidate t between 0 and a certain fixed maximum value:

The program module follows the above three steps to obtain a lumen_intima estimate corresponding to the threshold t. We refer to it as li(t)

The program module computes the mean squared error (MSE) between li(t) and the initial estimate of the lumen-intima interface computed in the steps of the first stage.

The program module selects the optimal $T^* = \min_t(MSE(t))$, so the threshold T* t for which the MSE was minimized [see Figure 9h: Graph displaying the MSE against the different values of the threshold t. The MSE is minimized at T*=20.].

**Example of Media - Adventitia Estimation**

According to one embodiment, the program module computes the far wall ROI y gradient map by applying a Sobel filter to the ROI image and denotes the gradient map [see Figure 9i: y gradient map after applying a Sobel filter to the raw far wall ROI image].

Before computing the score map, the program module computes the lumen-intimal basin mask, which we will refer to as B. Given the lumen-intima initial estimate computed in steps of the first stage, for each column:
The program module fills with 1s all pixels from the top to the first gradient local maxima after the lumen-intima position, and with 0's elsewhere, [see Figure 9j: Binary mask where 1's represents the lumen-intima basin. The red line represents the lumen-intima initial estimate.]

The program module computes a score map as follows:

$$C(x, y) = \begin{cases} G(x, y)(1 + W_{-} * (G(x, y) < 0) + \min(C(x-1, y+1), C(x-1, y), C(x-1, y-1)) ) \, if \, B(x, y) = 1 \\ 0 \qquad else \end{cases}$$

[see Figure 9k: Score map C(x,y)]

The program module retrieves the optimal path (path that maximizes the score along the map) by backtracking the increasing values in the score map. Note that between consecutive columns or pixels a maximum jump of 1 pixel in they direction is allowed, so that this is a strong "smooth" condition for the selected path. The optimal path will correspond to the media-adventitia interface [see Figure 9l: Score map C(x,y) with the optimal path corresponding to the media-adventitia display as a red line.].

The program module cleans the media-adventitia by removing the sides that have low quality. More specifically, the program module keeps the segment from the first to the last pixel whose gradient value is above a proportion of the gradient maxima along the media-adventitia [see Figure 9m: Left: gradient profile of the media-adventitia pixels. In red a threshold of 0.2 times the global maxima is displayed. Right: raw image with the final media-adventitia delineation displayed in red.].

Finally, the program module smooths both lumen-intima and media-adventitia delineations using a smoothing filter, for example a Savitzky-Golay filter [see Figure 9n: Left: Final lumen-intima and media-adventitia delineations for plaque. Right: Final smoothed lumen-intima and media- adventitia delineations for plaque].

## 7. Example of Image Quality Scoring

Image quality is critical for a good cIMT delineation and for good reproducibility of results. Therefore, a quality scoring system to quantify the quality of the image and ensure the system performs an analysis with good quality frames, may be provided.

A quality index is also displayed by the Philipps QLab software, as we can see in Figure 10 [see Figure 10: Image taken from Philipps QLab software, the QI=94 means that 94% of the points in the desired area were successfully delineated.]

According to some embodiments, a quality guidance scoring system (score in the range 0-100) may for example be provided as follows:

Lumen identification: from 0 to 15 based on the percentage of lumen that is detected in the image (linear).
Lumen horizontality: from 0 to 5 based on the angle (5 is perfectly horizontal, 0 if slope > 15%, values in between follow a linear relation).
Variability in far wall: from 0 to 10 based on how smooth the far wall is (10 is perfectly smooth, 0 if Mean Square Error (original-smoothed) > 35, values in between follow a linear relation).
Number of cutoff pieces of the sharpness quality delineation*: 0 to 30 (30 is 1, 0 if nb_pieces>100, values in between follow a linear relation).
Variability of the cIMT delineation: 0 to 10 - 5 for the lumen-intima and 5 for the media-adventitia. (5 if perfectly smooth, 0 if MSE (original-smoothed) > 35, values in between follow a linear relation).
Length of the cimt delineation: 0 to 30: 30 if the whole width of the image has been delineated, 0 if less than 1cm (recommended minimum length by Manheim Consensus guidelines), values in between follow a linear relation.
Thresholds to comply as best image:

If lumen identification percentage < 0.8, reject.
If absolute lumen horizontality (slope) > 0.15 , reject. If variability in far wall (MSE) > 35, reject.
If delineation length < 1cm, reject.
If the image passes all these thresholds one can consider the image as candidate for best image, and among these, the one with the highest score will win.

**8. Example of Sharpness Quality Delineation**

Image quality is critical for a good cIMT delineation and for good reproducibility of results. The program modules may include a quality scoring system to quantify the quality of the image and ensure that the systems performs analysis with good quality frames.

According to some embodiments, the goal of this algorithm is to delineate the lumen-intima and media-adventitia interfaces to provide an additional quality metric to our quality scoring system. The algorithm is very similar to the method proposed in [14] which may be adapted for this process.

Given a raw far wall ROI image as the one shown in Figure 1, the algorithm works as follows [see Figure 11a: Far wall ROI image]:
The program module computes a gaussian-smoothed version of the raw image, and the y gradient map of the raw image (e.g., using a Sobel filter for the latter) [see Figure 11b: Gradient map of the far wall ROI image].

For each column of the ROI:
The program module retrieves the profile of the y-gradient image, and the profile of the gaussian- smoothed version of the grayscale pixel intensity.

The program module locates the adventitia layer position as the maximum intensity point of the smoothed grayscale profile.

The program module identifies the two biggest significant peaks (local maxima) of the gradient located before the adventitia. A maximum is considered significant if its gradient is T times the maximum gradient value in the profile, where T is a fixed threshold [see Figure 11 c: Gradient profile of a fixed column, with lumen-intima and media-adventitia points marked in red.].

First and second peaks are identified with the lumen-intima and media- adventitia interfaces, respectively. If just one peak or no peaks are found the delineation for that column will be missing.

Finally, the program module retrieves the number of delineated pieces *nb_pieces* as the number of regions separated by missing delineated columns. Indeed, in a perfect quality image one would expect to see the lumen-intima and media-adventitia as two very sharp (high gradient) lines running along the image.

**9. Example of Plaque Identification**

The Manheim consensus [1] (2011) definition of plaque is the following:
*A focal structure that encroaches into the arterial lumen of at least 0.5 mm or 50% of the surrounding IMT value or demonstrates a thickness > 1.5 mm as measured from the media-adventitia interface to the intima-lumen interface.*

In the prior art, researchers typically use the second definition given by the Manheim consensus, to identify plaque if a cIMT-delineation region exceeds 1.5mm.

According to some embodiments, and given the raw far wall ROI image along with the delineated cIMT, the plaque location algorithm of the program modules according to an embodiment may work as follows:
The program module computes a median polyline distance between the lumen-intima and media adventitia, and uses such distance as baseline cIMT. It may be noted that the baseline cIMT is computed throughout the whole image except in the plaque region.

For each column, the program module computes the vertical distance between the lumen-intima point and the media-adventitia point thus obtaining a cIMT distance profile along the image.

All columns where the distance is above 1.5mm or above 150% of the baseline cIMT are labelled as plaque-region candidates. For each set of consecutive set of plaque-region candidates:
The program module computes the median cIMT in a window after and before the region. The minimum of both measures is defined as the neighboring cIMT If the maximum cIMT in the plaque-region candidate is below 150% of neighboring cIMT, the region is discarded as plaque candidate.

The program module merges all remaining plaque-region candidates that are closer than a defined distance, for example 2mm, and discards all plaque-region candidates whose length is lower than a second defined distance, for example 1.5mm

Finally for each plaque region, plaque pixels are those lying between the delineated lumen-intima and media-adventitia interfaces.

**10. Example of Plaque Characterization**

Plaque characterization is crucial for diagnosing cardiovascular diseases. Thus, once a plaque region is identified, one can retrieve several metrics that can eventually be used for cardiovascular risk assessment or stratification.

Note that for a full plaque characterization, and according to some embodiments, we require as input parameters the mean grayscale value of the identified lumen, and the mean grayscale value of the center of the adventitia layer. The retrieved metrics are the following [see Figure 12a: Far wall ROI raw image with the cIMT delineation in dark green and the plaque region located inside the light green bounding box.]

- Plaque length: the length of the plaque region is computed
- Plaque height: the maximum height of the plaque region is computed
- Plaque area: the number of pixels that lie inside the delineated plaque region
- Plaque composition is computed:

According to some embodiments, the program module first linearly normalizes all pixel values with two references: the lumen grayscale median should correspond to 0 and the adventitia grayscale median is 180. Thus, each pixel value x is transformed to x' as follows:

$$0 \ \textit{if } x < 0$$

$$(x - l_{gsm})/(adv_{gsm} - l_{gsm}) * 180 \quad \textit{if} \quad 0 <= x < adv_{gsm}$$

$$180 + ((x - adv_{gsm})/(255 - adv_{gsm})) * (255 - 180) \quad \textit{if} \quad x >= adv_{gsm} 0$$

Each pixel may be classified into type of tissue (blood, lipid, muscle, fibrous, calcium or connective) according to their normalized intensity values

*Blood if* $0 <= x' <= 4$
*lipid if* $8 <= x' <= 26$
*muscle if* $41 <= x' <= 76$
*fibrous if* $112 <= x' <= 196$ *calcium if* $211 <= x' <= 255$ *connective else* [see Figure 12b: Plaque composition according to pixel grayscale intensities]

## 11. and 12. Examples of Wall Tracking and Waveform Processors

Note that details and examples regarding data processing blocks 11 (Wall Tracker) and 12 (Waveform Processor) are set forth below in additional slides and Figures.

## Some Examples of Image Processing and Machine Learning Tools That May Be Used in the Different Data Processing Blocks

1. Transverse Carotid and Bifurcation Identification

- CNN's (Deep Learning)
- YOLOv5 model (Deep Learning)

2. Frame Cropping

- Otsu Thresholding
- Morphological (closing, opening) operations

3. Lumen Delineation

- Gaussian smoothing
- Min-max scaling (normalization technique)

4. Far Wall ROI Segmentation

- Otsu Thresholding

5. cIMT Delineation

- Gaussian smoothing
- Sobel filter

6. Plaque Delineation

- Gaussian smoothing
- Sobel filter

7. Image Quality
8. Sharpness Quality Delineation

- Gaussian smoothing
- Sobel filter

9. Plaque Identification

- Various processing modules

10. Plaque Characterization

- Various processing modules

11. Wall Tracker

- Gaussian filter
- Gradient of gaussian filter

12. Waveform Processor

- Fast Fourier Transform
- Band-pass FIR filter

**Some Other Filtering and Edge Detection Techniques that May Be Used in, For Example, Data Processing Blocks 3,5,6,8,10**

- Canny edge detector
- Laplacian edge detector
- ICOV filter
- FOAM edge detector
- Anisotropic filtering
- Homomorphic filtering
- Median filter

Further details and information regarding the various embodiments are set forth below in the numerous Figures and slides attached hereto, including slides describing and disclosing further details regarding steps 11 and 12 (Wall Tracker and Waveform Processor, respectively). See also Fig. 13, which shows a flow chart corresponding to another embodiment of an ultrasound image processing system for cardiovascular health assessment based on ultrasound images of a patient's carotid artery according to one embodiment of the invention.

Described above are various embodiments of methods and systems relating to detecting and measuring various physical characteristics and properties associated with vascular systems such as the carotid arteries of a patient. Many of these embodiments work well in an environment where elongated or tubularly-shaped anatomical organs such as vasculature of various types, arteries, veins, nerves and/or portions of gall bladders, are the object of the detection and measurement. However, at least some of the systems and methods described and disclosed herein may also be adapted or modified to permit the detection and measurement of various physical characteristics and properties of anatomical organs that are generally not elongated or tubularly shaped, such as kidneys, livers, lungs, the human heart or portions thereof, the human brain, and so on.

In other words, some of the various embodiments described and disclosed herein are not limited to carotid artery applications, and in addition to including the applications listed above in this paragraph and elsewhere herein, also find application in detecting and measuring various physical characteristics and properties associated with the femoral arteries, the aorta, the Circle of Willis in the human brain, interior and exterior carotid arteries, and so on.

Some examples of arteries that can be detected and measured according to various the embodiments disclosed and described herein include, but are not limited to, the brachiocephalic trunk artery, the subclavian artery, the common carotid artery, the external carotid artery, the internal carotid arteries, the thoracic aorta, the abdominal aorta, the iliac arteries, the axillary arteries, the brachial arteries, the ulnar artery, the radial arteries, the femoral artery, the popliteal artery, the anterior tibial artery, the posterior tibial artery, the dorsalis pedis arteries, the anterior cerebral artery, the anterior communicating artery, the middle cerebral artery, the posterior communicating artery, the posterior cerebral artery, the basilar artery, the vertebral artery, the posterior inferior cerebral artery, and any other suitable artery.

Some examples of veins that can be detected and measured according to various the embodiments disclosed and described herein include, but are not limited to, the internal jugular vein, the external jugular vein, the anterior jugular vein, the subclavian vein, the brachiocephalic vein, the superior vena cava, the azygos vein, the iliac veins, the inferior vena cava, the basilic vein, the cephalic vein, the radial vein, the ulnar vein, the brachial vein, the axillary vein, the anterior tibial vein, the posterior tibial vein, the fibular/peroneal vein, the popliteal vein, the femoral vein, the great saphenous vein, the small saphenous vein, the external iliac vein, the common iliac veins and any other suitable vein.

Additional regions of the human body that can be detected and measured that are more distantly related to the existing technology described and disclosed herein, but which would benefit from a guidance system provided in accordance with the embodiments , include, but are not limited to, bone structures, internal organs, abdominal organs, muscles, nerves, ligaments, fat tissues, connective tissues, the eyes, the brain, the skin, and pathologies such as tumors, cysts, and any other similar pathology. Procedural guidance for syringe detection and guidance and biopsy detection and guidance can also be provided in accordance with some embodiments.

In addition, the various embodiments are not limited to the use of B-mode ultrasound devices or M-mode ultrasound devices, but may also include RF-mode ultrasound devices (which can permit higher-resolution images to be acquired) and doppler-mode ultrasound devices (which, by way of example, can permit the velocity or speed of blood flow to be measured).

The data processing modules described and disclosed above may be modified individually or together to permit the use of machine learning or artificial intelligence techniques in addition to those described above.

The computing and display devices employed in the various embodiments described and disclosed herein may or may not be portable, employ cloud-based analytics and/or data transfer and distribution, be web-based, employ a cell phone or iPhone, a tablet, a laptop, or a desktop computer, operate on-line or off-line, and so on.

What have been described above are examples and embodiments of the systems, devices, components and methods described and disclosed herein. It is, of course, not possible to describe every conceivable combination of components or methodologies for purposes of describing the invention, but one of ordinary skill in the art will recognize that many further combinations and permutations of the devices and methods described and disclosed herein are possible. Accordingly, the devices and methods described and disclosed herein are intended to embrace all such alterations, modifications and variations that fall within the scope of the appended claims.

In the claims, unless otherwise indicated, the article "a" is to refer to "one or more than one."

The foregoing outlines features of several embodiments so that those skilled in the art may better understand the detailed description set forth herein. Those skilled in the art will now understand that many different permutations, combinations and variations of the systems and methods described herein fall within the scope of the various embodiments. Those skilled in the art should appreciate that they may readily use the present disclosure as a basis for designing or modifying other processes and structures for carrying out the same purposes and/or achieving the same advantages of the embodiments introduced herein.

Those skilled in the art should also realize that such equivalent constructions do not depart from the spirit and scope of the present disclosure, and that they may make various changes, substitutions and alterations herein without departing from the spirit and scope of the present disclosure.

After having read and understood the present specification, those skilled in the art will now understand and appreciate that the various embodiments described herein provide solutions to long-standing problems in measuring and quantifying the state of health of a patient's carotid arteries quickly, accurately, and economically.

**List of Selected Feature References**

B-mode ultrasound image processing system for measurement of cardiovascular parameters

Ultrasound probe device
Blood pressure measurement device
Computing system
Output device (e.g., computer, smartphone, smartpad)
Graphical user interface
Program modules

    Image capture assistant
    Lumen and ROI detection
    cIMT measurement
    plaque identification

Databases
Image database (still, moving)
→ (for reference, for training)
Plaque reference database
cIMT reference database
stiffness reference database

**Literature References**

[1] Automatic localization of Common Carotid Artery in ultrasound images using Deep Learning Dina A. Hassanin, Mahmoud khaled Abd-Ellah, Ashraf A. M. Khalaf and Reda R. Gharieb 2021

[2] Localization of common carotid artery transverse section in B-mode ultrasound images using faster RCNN: a deep learning approach Pankaj K. Jain. Saurabh Gupta. Arnav Bhavsar. Aditya Nigam, Neeraj Sharma 2020

[3] Automatic segmentation of carotid B-mode images using fuzzy classification Rui Rocha, Jorge Silva, Aurélio Campilho 2011

[4] A Theshold Selection Method from Gray-Level Histograms Nobuyuki Otsu 1979

[5] Automatic detection of the carotid lumen axis in B-mode ultrasound images Rui Rochaa, Jorge Silvaa, Aurélio Campilho 2013

[6] Characterization of a Completely User- Independent Algorithm for Carotid Artery Segmentation in 2-D Ultrasound Images Silvia Delsanto, Filippo Molinari, Pierangela Giustetto, William Liboni,Sergio Badalamenti, and Jasjit S. Suri 2007

[7] Automatic Computer-based Tracings (ACT) in longitudinal 2-D ultrasound images using different scanners Filippo Molinari, William Liboni, Pierangela Giustetto, Sergio Badalamenti, Jasit. S Suri 2009

[8] Intima-media thickness: setting a standard for a completely automated method of ultrasound measurement Filippo Molinari; Guang Zeng; Jasjit S. Suri 2010

[9] A Theshold Selection Method from Gray-Level Histograms Nobuyuki Otsu 1979

[10] Intima-media thickness: setting a standard for a completely automated method of ultrasound measurement Filippo Molinari; Guang Zeng; Jasjit S. Suri 2010

[11] A Fully-Automatic Method to Segment the Carotid Artery Layers in Ultrasound Imaging: Application to Quantify the Compression-Decompression Pattern of the Intima-Media Complex During the Cardiac Cycle Guillaume Zahnd, Kostas Kapellas, Martijn Van Hattem, ANouk Van Dijk 2017

[12] A Fully-Automatic Method to Segment the Carotid Artery Layers in Ultrasound Imaging: Application to Quantify the Compression-Decompression Pattern of the Intima-Media Complex During the Cardiac Cycle Guillaume Zahnd, Kostas Kapellas, Martijn Van Hattem, ANouk Van Dijk 2017

[13] Segmentation of the carotid intima-media region in B-mode ultrasound images Rui Rocha, Aurélio Campilho, Jorge Silva, Elsa Azevedo, Rosa Santos 2010

[14] Real-time measurement system for the evaluation of the Intima Media Thickness with a new edge detector Francesco Faita, Vincenzo Gemignani, Elisabetta Bianchini, Chiara Giannarelli, and Marcello Demi 2006

[15] Mannheim Carotid Intima-Media Thickness and Plaque Consensus (2004-2006-2011) P.J. Touboul, M.G. Hennerici, S. Meairs, H. Adams, P. Amarenco, N. Bornstein, L. Csiba, M. Desvarieux, S. Ebrahim R. Hernandez, M. Jaff, S. Kownator, T. Naqvi, P. Prati, T. Rundek, M. Sitzer, U. Schminke, J.-C. Tardif, A. Taylor, E. Vicaut, K.S. Woo

**Claims**

1. A system configured to rapidly measure, analyze and provide, in an on-site or out-patient setting and within a predetermined period of time, one or more physical parameters or characteristics associated with a one or more of a patient's carotid arteries, the system comprising:

    (a) at least one computing device;
    (b) at least one portable ultrasound device or probe operably connected to the at least one computing device, the portable ultrasound device being configured to provide to the computing device as outputs therefrom a series of ultrasound image frames acquired from the one or more of the patient's carotid arteries, and
    (c) a display or monitor operably connected to the at least one computing device and configured to visually display to a user results generated by the at least one computing device;

    wherein the computing device comprises at least one non-transitory computer readable medium configured to store instructions executable by at least one processor to process the ultrasound image frames, the at least one processor and the at least one non-transitory computer readable medium further being configured to process the ultrasound image frames using a trained discriminative or convolutional machine learning model, the computing device and ultrasound device or probe being configured to:

    (i) identify at least one of a bifurcation, a transverse view, and a cross-sectional view of at least one of the patient's carotid arteries from among the ultrasound image frames generated by the ultrasound device or probe;
    (ii) generate directional and locational instructions to the user on the display or monitor or via sound regarding the location, placement, orientation and movement of the ultrasound device or probe;
    (iii) measure and compute at least one of lumen distention, carotid intima-media thickness (cIMT), cardiovascular age, plaque characterization, and local arterial stiffness of the at least one carotid artery; and
    (iv) provide as outputs from the computing device at least one of the measured and computed lumen distention, the measured and computed carotid intima-media thickness (cIMT), the measured and computed cardiovascular age, the measured and computed plaque characterization, and the measured and computed local arterial stiffness of the at least one carotid artery to one or more of the monitor or display, a printer, a speaker or headphones, or data formatted for digital or memory storage or transmission.

2. The system of claim 1, wherein the ultrasound device or probe is configured to switchably and controllably operate in B-mode or M-mode, preferably wherein the ultrasound device or probe is configured to switchably and controllably operate in the M-mode when measuring lumen distention.

3. The system of claim 1, wherein the trained discriminative or convolutional machine learning model is trained and configured to generate directional and locational instructions to the user on the display or monitor or via sound regarding at least one of the location, placement, orientation and movement of the ultrasound device or probe to identify one or more of carotid artery bifurcation locations, carotid artery cross-sectional characteristics, carotid artery plaque characteristics, carotid artery lumen characteristics, carotid artery regions of interest, and carotid artery far wall variability, preferably wherein the trained discriminative or convolutional machine learning model is trained and configured to provide directional and locational instructions to the user regarding whether the ultrasound probe or device is centered over the patient's carotid artery.

4. The system of claim 1, wherein lumen distention is measured and computed using the patient's blood pressure as an input.

5. The system of claim 1, wherein the trained discriminative or convolutional machine learning model is trained and configured to:

crop the ultrasound image frames so as to enhance reliability of the detection and identification one or more of carotid artery bifurcation locations, carotid artery cross-sectional characteristics, carotid artery plaque characteristics, carotid artery lumen characteristics, carotid artery regions of interest, and carotid artery far wall variability, and/or

determine the quality of the ultrasound image frames so as to enhance reliability of the detection and identification one or more of carotid artery bifurcation locations, carotid artery cross-sectional characteristics, carotid artery plaque characteristics, carotid artery lumen characteristics, carotid artery regions of interest, and carotid artery far wall variability.

6. The system of claim 1, wherein the trained discriminative or convolutional machine learning model is trained and configured to measure and compute one or more of the number, location, length, height, area and echogenicity of each plaque in the at least one carotid artery as part of the plaque characterization.

7. The system of claim 1, wherein the predetermined period of time is about ten minutes or less.

8. A system for processing brightness mode ultrasound images in medical applications, in particular for measurement of cardiovascular parameters, comprising a computing system and program modules executable in the computing system configured to process data generated by a B-mode ultrasound probe device of a patient's carotid artery, the program modules include modules for processing an image generated by the B-mode ultrasound probe device, including automated frame cropping of said image, lumen delineation, far wall ROI segmentation and cIMT delineation, wherein the lumen delineation module is configured to:

divide said image in a plurality of columns extending from a first side of the image captured closest to the ultrasound probe device to a second opposite side of the image captured furthest from the ultrasound probe device;

determine in each column at least a first and a last significant local intensity maxima 11, I2, I3 of image pixels above a chosen fixed intensity threshold;

determine in each column at least one significant local intensity minima i1, i2 of image pixels between the first significant local intensity maxima I1 and the last significant local intensity maxima I3;

compute paths of connected pixels from said local significant local intensity minima i1, i2 within a predefined distance from the significant local intensity maxima;

calculate the lengths of the paths;

select paths having a length greater than a fixed proportion of a path with the greatest length, said fixed proportion in a range of 0,7 to 0,95, and

define a lumen axis as the selected path furthest from the first side of the image.

9. The system according to the claim 8, wherein the intensity value of image pixels are normalized in a fixed scale, for example from 0 to 1, preferably wherein fixed intensity threshold is in a range of 0.1 to 0.3 of the fixed scale, preferably in a range of 0.15 to 0.25.

10. The system according to one of claims 8 and 9, wherein three said significant local intensity maxima I1, I2, I3 of image pixels above a chosen fixed intensity threshold are determined, and two said significant local intensity minima i1, i2 are determined.

11. The system according to one of claims 8 to 10, wherein said predefined distance from the significant local intensity maxima falls within a predetermined range.

12. The system according to one of claims 8 to 11, wherein the frame cropping module is configured to generate a binary mask in which pixels whose intensity values lie between a first intensity threshold T1 and a second intensity threshold T2 are set to bright, and pixels having an intensity below the first threshold and above the second threshold are set to dark,

and wherein the frame cropping or lumen delineation module is configured to apply a soothing filter, for example a gaussian smoothing filter, to the binary mask for further processing by the lumen delineation module.

13. The system according to one of claims 8 to 12, wherein the far wall ROI segmentation and cIMT delineation modules are configured to generate a binary mask of the image after the lumen delineation processing in which a threshold T is computed to separate pixels into two classes according to their brightness intensity:

    - pixels having intensities below the threshold set to dark pixels and corresponding to the lumen;
    - pixels having intensities above the threshold set to bright and corresponding to

the wall interfaces below the lumen and other hyperechoic tissues;
and identify a far wall line of the artery in columns extending from the first side of the image to the second opposite side of the image, as the first line of bright pixels extending laterally across the columns.

14. The system according to claim 13, wherein the far wall ROI segmentation and cIMT delineation modules are configured to

    - smooth the far wall line with a smoothing filter, for example a Savitzky-Golay filter, and
    - define upper and lower bounds of a far wall ROI with a predefined height parameter to generate a far wall ROI image, preferably

wherein the cIMT delineation module is configured to

    - apply a filter, for example a gaussian filter, on the far wall ROI image to reduce speckle noise and generate a smoothed intensity map,
    - apply a Sobel filter on the smoothed intensity map in the column direction $y$ to retrieve a $y$-gradient map of a region of interest,
    - retrieved continuous paths passing through maximum intensity values defined as a center of adventitia layer,
    - retrieve all gradient local maxima located above the adventitia center,
    - select a pre-defined number of the longest paths and retaining overlapping paths, which are considered as lumen-intima or media-adventitia candidates, the paths located closest to the first side being identified as lumen-intima, and the paths closest to the second side being identified as the media-adventitia,
    - defining as ROI a section having a pair of paths with the largest overlap.

15. The system according to one of claims 8 to 14, wherein the program modules comprise a transverse carotid and bifurcation identification module to provide visual feedback to assist a medical practitioner in identifying a carotid artery and a bifurcation of the carotid artery, the transverse carotid and bifurcation identification module configured to identify and mark with a bounding box the carotid in a transverse B-mode ultrasound image, and identify the bifurcation if a ratio of a length to height of the bounding box is above a certain fixed threshold.

Fig. 1

Fig. 2A

Fig. 2B

Fig. 2C

Fig. 2D

Fig. 2E

Fig. 3A

Fig. 3B

Fig. 4A

Fig. 4B

Fig. 4C

30

Fig. 5A

Fig. 5B

Fig. 5C

1st side- closest to ultrasound probe

2nd side- furthest from ultrasound probe

Sternocleidomastoid muscle

Jugular vein

Common carotid artery

Lumen

Intima-media

Fig. 6A

Fig. 6B

column

I1
i1
I2
i2
I3

Fig. 6C

Fig. 6E

Fig. 6D

Fig. 6G

Fig. 6F

Fig. 6H

Fig. 7A

Fig. 7B

Fig. 7C

Fig. 7D

Fig. 7F

Fig. 7E

Fig. 7G

Fig. 8A

Fig. 8B

Fig. 8C

Fig. 8D

Fig. 8E

Fig. 8F

EP 4 338 682 A2

Fig. 9A

Fig. 9B

Fig. 9C

Fig. 9D

Fig. 9E

Fig. 9F

Fig. 9G

Fig. 9H

Fig. 9I

Fig. 9J

Fig. 9K

Fig. 9L

Fig. 9M

Fig. 9N

EP 4 338 682 A2

QI 94          Maximal: 0,788mm          Mean: 0,625mm

Fig. 10

Fig. 11A

Fig. 11B

Fig. 11C

1 plaques found.

Fig. 12A

Blood
Lipid
Muscle
Fibrous
Calcium
Connective

Fig. 12B

Fig. 13

Fig. 13 (continued)

Fig. 13 (continued)

| | Hardware | | Process/method |
| --- | --- | --- | --- |
| | Guidance | | Object |
| | CIMT and plaque | | Decision |
| | Stiffness | | Data file |
| | External source | | Data files |
| | Output | | Database |

Fig. 13 (continued)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **DINA A. HASSANIN ; MAHMOUD KHALED ABD-ELLAH ; ASHRAF A. M. KHALAF ; REDA R. GHARIEB.** *Automatic localization of Common Carotid Artery in ultrasound images using Deep Learning,* 2021
- **PANKAJ K. JAIN. ; SAURABH GUPTA. ; ARNAV BHAVSAR. ; ADITYA NIGAM ; NEERAJ SHARMA.** *Localization of common carotid artery transverse section in B-mode ultrasound images using faster RCNN: a deep learning approach,* 2020
- **RUI ROCHA ; JORGE SILVA ; AURÉLIO CAMPILHO.** *Automatic segmentation of carotid B-mode images using fuzzy classification,* 2011
- **NOBUYUKI OTSU.** *A Theshold Selection Method from Gray-Level Histograms,* 1979
- **RUI ROCHAA ; JORGE SILVAA ; AURÉLIO CAMPILHO.** *Automatic detection of the carotid lumen axis in B-mode ultrasound images,* 2013
- **SILVIA DELSANTO ; FILIPPO MOLINARI ; PIERANGELA GIUSTETTO ; WILLIAM LIBONI ; SERGIO BADALAMENTI ; JASJIT S. SURI.** *Characterization of a Completely User- Independent Algorithm for Carotid Artery Segmentation in 2-D Ultrasound Images,* 2007
- **FILIPPO MOLINARI ; WILLIAM LIBONI ; PIERANGELA GIUSTETTO ; SERGIO BADALAMENTI ; JASIT. S SURI.** *Automatic Computer-based Tracings (ACT) in longitudinal 2-D ultrasound images using different scanners,* 2009
- **FILIPPO MOLINARI ; GUANG ZENG ; JASJIT S. SURI.** *Intima-media thickness: setting a standard for a completely automated method of ultrasound measurement,* 2010
- **GUILLAUME ZAHND ; KOSTAS KAPELLAS ; MARTIJN VAN HATTEM ; ANOUK VAN DIJK.** *A Fully-Automatic Method to Segment the Carotid Artery Layers in Ultrasound Imaging: Application to Quantify the Compression-Decompression Pattern of the Intima-Media Complex During the Cardiac Cycle,* 2017
- **RUI ROCHA ; AURÉLIO CAMPILHO ; JORGE SILVA ; ELSA AZEVEDO ; ROSA SANTOS.** *Segmentation of the carotid intima-media region in B-mode ultrasound images,* 2010
- **FRANCESCO FAITA ; VINCENZO GEMIGNANI ; ELISABETTA BIANCHINI ; CHIARA GIANNARELLI ; MARCELLO DEMI.** *Real-time measurement system for the evaluation of the Intima Media Thickness with a new edge detector,* 2006
- **P.J. TOUBOUL ; M.G. HENNERICI ; S. MEAIRS ; H. ADAMS ; P. AMARENCO ; N. BORNSTEIN ; L. CSIBA ; M. DESVARIEUX ; S. EBRAHIM ; R. HERNANDEZ.** *Mannheim Carotid Intima-Media Thickness and Plaque Consensus,* 2004